# EUROPEAN PATENT SPECIFICATION

(11) **EP 3 841 963 B1**
(45) Date of publication and mention of the grant of the patent: **24.05.2023**
(21) Application number: 20195361.9
(22) Date of filing: 09.09.2020
(51) Int. Cl.: A61B 5/00, A61B 5/145, A61B 5/15

(54) **WEARABLE SENSING DEVICE**
ERFASSENDE WEARABLE-VORRICHTUNG
DISPOSITIF DE DÉTECTION VESTIMENTAIRE

(30) Priority: 27.12.2019 US 201962953997 P
(43) Date of publication of application: 30.06.2021
(73) Proprietor: RichHealth Technology Corporation, Zhubei City 302 (TW)
(72) Inventor: LEE, Chih-Ta, 302 Hsinchu County (TW); WANG, Shy-Horng, 302 Hsinchu County (TW)
(74) Representative: Yang, Shu

(56) References cited:
- WO-A1-2016/009228
- US-A1- 2002 188 184
- US-A1- 2015 208 985
- US-A1- 2017 086 713

## Description

### CROSS-REFERENCES TO RELATED APPLICATIONS

This application claims the benefit of U.S. Provisional Patent Application No. 62953997 filed on Dec. 27, 2019.

### Background

### 1. Field

The present disclosure generally relates to wearable sensing device, and more particularly pertains to wearable physiological sensing device having piercers that penetrate the wearer's skin for obtaining biometric measurements.

### 2. Related Art

Point of care testing refers to testing performed in a healthcare setting that's not done in a lab. There is an on-going trend to bring healthcare related testing closer to a patient. Quick and reliable diagnosis would be beneficial to patient and care-taker alike by providing on-time and on-set healthcare outcomes. Wearable sensing devices are a growing type of point of care testing device that can provide quick and reliable monitoring/diagnosis for a patient.
US2015208985A1 discloses a transdermal microneedle array patch for measuring a concentration of a hypodermal target molecule. The transdermal microneedle array patch includes a substrate, a microneedle unit, a signal processing unit and a power supply unit. The microneedle unit at least comprises a first microneedle set used as a working electrode and a second microneedle set used as a reference electrode, the first and second microneedle sets arranging on the substrate. Each microneedle set comprises at least a microneedle. The first microneedle set comprises at least a sheet having a through hole on which a barbule forms at the edge. One of the sheet provides the through hole from which the barbules at the edge of the other sheets go through, and the barbules are disposed separately.
US2017086713A1 discloses a system and method for monitoring body chemistry of a user, the system comprising: a housing supporting: a microsensor comprising a first and second working electrode, a reference electrode, and a counter electrode, and configured to access interstitial fluid of the user, and an electronics subsystem comprising a signal conditioning module that receives a signal stream, from the microsensor, wherein the electronics subsystem is configured to detect an impedance signal derived from two of the first working electrode, the second working electrode, the reference electrode, and the counter electrode; and a processing subsystem comprising: a first module configured to generate an analysis indicative of an analyte parameter of the user and derived from the signal stream and the impedance signal, and a second module configured to transmit information derived from the analysis to the user, thereby facilitating monitoring of body chemistry of the user. WO2016009228A1 discloses a microstructured electrode array comprising a base with a plurality of groups of probes formed thereon, each group of probes having a conductive layer formed thereon so that the group forms a single electrode, wherein the electrodes are electrically isolated from each other.
US2002188184A1 discloses a device for sampling a biological fluid and measuring at least one target constituent within the biological fluid. The device has at least one electrochemical cell having an inner electrode and an outer electrode in a concentrically-spaced relationship. In a preferred embodiment, the outer electrode has a cylindrical configuration having an open distal end and the inner electrode has an elongated configuration positioned coaxially within the outer electrode and a distal end configured to penetrate the skin. The spacing between the electrodes exerts a capillary force on biological fluid present at the open distal end of the outer electrode. A system is also provided which includes a control unit in electrical communication with the electrochemical cell for controlling the selection and measurement of the target constituent. Methods of sampling of biological fluids within the skin and measuring the sampled fluids are also provided, as well as kits comprising one or more of the inventive devices and/or systems.

### Brief Description of the Drawings

So that the manner in which the above recited features of the present disclosure can be understood in detail, a more particular description of the disclosure, briefly summarized above, may be had by reference to embodiments, some of which are illustrated in the appended drawings. It is to be noted, however, that the appended drawings illustrate only typical embodiments of this disclosure and are therefore not to be considered limiting of its scope, for the disclosure may admit to other equally effective embodiments.
FIG. 1 illustrates a sectional exploded view of a wearable sensing device according to some embodiments of the present disclosure;
FIG.2 illustrates a planar view of a user interface module of a wearable sensing device according to some embodiments of the present disclosure;
FIG.3 illustrates a planar view of a user interface module of a wearable sensing device according to some embodiments of the present disclosure;
FIG.4 illustrates a planar view of a user interface module of a wearable sensing device according to some embodiments of the present disclosure;
FIG.5 illustrates a planar view of a user interface module of a wearable sensing device according to some embodiments of the present disclosure;
FIG.6 illustrates a planar view of a user interface module of a wearable sensing device according to some embodiments of the present disclosure;
FIG.7 illustrates a planar view of a user interface module of a wearable sensing device according to some embodiments of the present disclosure;
FIG.8 illustrates a planar view of a user interface module of a wearable sensing device according to some embodiments of the present disclosure;
FIG.9 illustrates a planar view of a user interface module of a wearable sensing device according to some embodiments of the present disclosure;
FIG. 10 illustrates a planar view of a user interface module of a wearable sensing device according to some embodiments of the present disclosure;
FIG. 11 illustrates a planar view of a user interface module of a wearable sensing device according to some embodiments of the present disclosure;
FIG.12A illustrates a planar view of a microprobe for a wearable sensing device according to some embodiments of the present disclosure;
FIG.12B illustrates a planar view of a microprobe for a wearable sensing device according to some embodiments of the present disclosure;
FIG. 12C illustrates a sectional view of a microprobe for a wearable sensing device according to some embodiments of the present disclosure;
FIG. 13A illustrates a planar view of a microprobe for a wearable sensing device according to some embodiments of the present disclosure;
FIG.13B illustrates a planar view of a microprobe for a wearable sensing device according to some embodiments of the present disclosure;
FIG.13C illustrates a sectional view of a microprobe for a wearable sensing device according to some embodiments of the present disclosure;
FIG.14A illustrates a planar view of a microprobe for a wearable sensing device according to some embodiments of the present disclosure;
FIG.14B illustrates a planar view of a microprobe for a wearable sensing device according to some embodiments of the present disclosure;
FIG. 14C illustrates a sectional view of a microprobe for a wearable sensing device according to some embodiments of the present disclosure;
FIG.15A illustrates a planar view of a microprobe for a wearable sensing device according to some embodiments of the present disclosure;
FIG.15B illustrates a planar view of a microprobe for a wearable sensing device according to some embodiments of the present disclosure;
FIG. 15C illustrates a sectional view of a microprobe for a wearable sensing device according to some embodiments of the present disclosure;
FIG.16A illustrates a planar view of a microprobe for a wearable sensing device according to some embodiments of the present disclosure;
FIG.16B illustrates a planar view of a microprobe for a wearable sensing device according to some embodiments of the present disclosure;
FIG. 16C illustrates a sectional view of a microprobe for a wearable sensing device according to some embodiments of the present disclosure;
FIG.17A illustrates a planar view of a microprobe for a wearable sensing device according to some embodiments of the present disclosure;
FIG.17B illustrates a planar view of a microprobe for a wearable sensing device according to some embodiments of the present disclosure;
FIG. 17C illustrates a sectional view of a microprobe for a wearable sensing device according to some embodiments of the present disclosure;
FIG.18A illustrates a planar view of a microprobe for a wearable sensing device according to some embodiments of the present disclosure;
FIG.18B illustrates a planar view of a microprobe for a wearable sensing device according to some embodiments of the present disclosure;
FIG. 18C illustrates a sectional view of a microprobe for a wearable sensing device according to some embodiments of the present disclosure;
FIG.19A illustrates a planar view of a microprobe for a wearable sensing device according to some embodiments of the present disclosure;
FIG.19B illustrates a planar view of a microprobe for a wearable sensing device according to some embodiments of the present disclosure;
FIG. 19C illustrates a sectional view of a microprobe for a wearable sensing device according to some embodiments of the present disclosure;
FIG.20A illustrates a planar view of a microprobe for a wearable sensing device according to some embodiments of the present disclosure;
FIG.20B illustrates a planar view of a microprobe for a wearable sensing device according to some embodiments of the present disclosure;
FIG.20C illustrates a planar view of a group of microprobes for a wearable sensing device according to some embodiments of the present disclosure; and
FIG.21 illustrates a system module block diagram for a wearable sensing device according to some embodiments of the present disclosure.

### Detailed Description

The present disclosure will now be described more fully hereinafter with reference to the accompanying drawings, in which exemplary embodiments of the disclosure are shown. This disclosure may, however, be embodied in many different forms and should not be construed as limited to the exemplary embodiments set fourth herein. Rather, these exemplary embodiments are provided so that this disclosure will be thorough and complete, and will fully convey the scope of the disclosure to those skilled in the art. Like reference numerals refer to like elements throughout.

The terminology used herein is for the purpose of describing particular exemplary embodiments only and is not intended to be limiting of the disclosure. As used herein, the singular forms "a", "an" and "the" are intended to include the plural forms as well, unless the context clearly indicates otherwise. It will be further understood that the terms "comprises" and/or "comprising," or "includes" and/or "including" or "has" and/or "having" when used herein, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

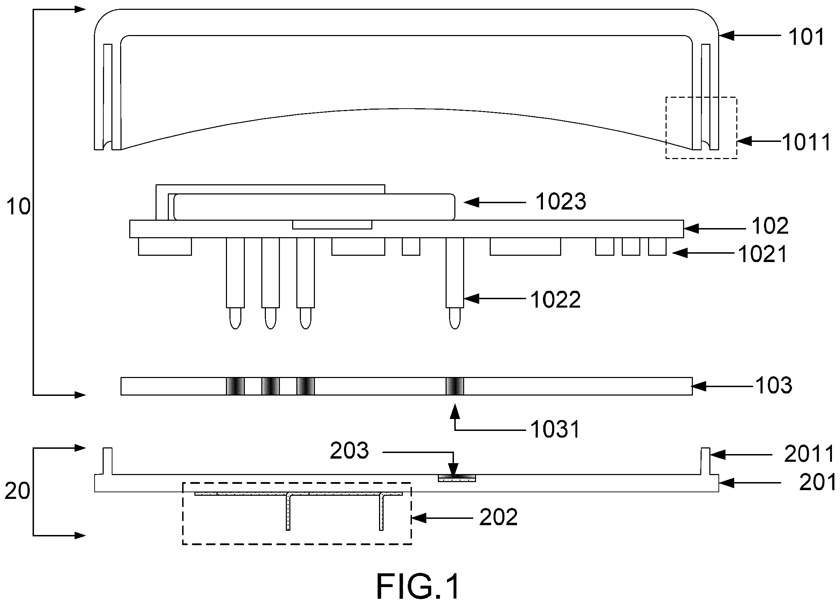

FIG. 1 illustrates a sectional view of a wearable sensing device according to some embodiments of the present disclosure. The wearable sensing device may be configured to monitor an analyte found in the interstitial fluid of a wearer. Depending on practical applications, the analyte monitored by the exemplary wearable sensing device may include, for example, glucose, acetyl choline, amylase, bilirubin, cholesterol, chorionic gonadotropin, creatine kinase (e.g., CK-MB), creatine, DNA, fructosamine, glutamine, growth hormones, hormones, ketones, lactate, peroxide, prostate-specific antigen, prothrombin, RNA, thyroid stimulating hormone, and troponin.

The analyte mentioned above may often be found in the biofluid (e.g., blood or interstitial fluid) under the skin of a test subject whose biometric status is to be monitored. The biometric information associated with the analyte are typically extracted invasively, e.g., through the use of insertion needles. In many applications, longer needles are used (e.g., with length in a range of 4mm to 8mm, which would penetrate the skin and reach the deeper subcutaneous space region) to ensure reliable access to the analyte. However, wearable sensor devices with long insertion needle are often intimidating, and may inflict greater pain and discomfort upon application.

There has been a trend to incorporate microprobes (e.g., micro-piercers) with shorter length. However, shorter needle length inevitably reduces probe efficacy and reliability (e.g., both in terms of piercer retention and sensing efficiency). To compensate for potential shortcomings of shorter piercer length, an array of multiple probes may be used to increase overall contact area with transdermal biofluid.

The exemplary wearable sensing device includes a function module 10 and a user interface module 20. The function module 10 may encompass a plurality of active functional components (e.g., power supply unit, circuit components, processing units, etc.). For instance, the illustrated function module 10 includes a top portion 101, a bottom portion 103, and a substrate 102 disposed between the top portion 101 and the bottom portion 103.

In some embodiments, the substrate 102 may be a non-conductive board having conductive materials such copper laminated thereon to form conductive tracks, pads and other features. Electronic components 1021, a battery 1023, and conductive posts 1022 are disposed on the substrate 102. The electronic components 1021, the battery 1023, and the conductive posts 1022 may be electrically connected to each other through the conductive tracks of the substrate 102.

In some embodiments, the electronic components 1021 include transmitter circuit components configured to transmit information gathered by the microprobes (e.g., probes 202) to an off-board device. In some embodiments, the electronic components 1021 may include readout circuit components configured to convert detected biometric signals to equivalent electrical signals such as voltage, current, or resistance. Battery 1023 is provided to power the electronic components 1021.

The conductive posts 1022 are provided to establish electrical coupling between the function module 10 and the user interface module 20. In some embodiments, the conductive posts 1022 are spring loaded pins (e.g., pogo pins) configured to exert normal force onto the user interface module 20 to ensure continuous electrical coupling while the device wearer is sedentary or in motion. In some embodiments, the conductive posts 1022 are configured to protrude from the bottom portion 103. For instance, the conductive posts 1022 may pass through through-holes 1031 from one side of the bottom portion 103 and reach the opposite face of bottom portion 103. The position of the conductive posts 1022 on the substrate 102 may be arranged in substantial alignment with respect to the through-holes 1031 of the bottom portion 103.

The user interface module 20 incorporates a separable modular design (e.g., detachably coupled to the functional module 10) and comprises the probing interface of the various functional electrodes that come into contact with a device wearer. The exemplary user interface module 20 includes a carrier 201, a plurality of electrode probes 202 disposed on a contact face 2013 (e.g., the downward facing surface) of the carrier 201, and a switch plate 203 disposed on a back face of the carrier 201. The separable interface module design offers several beneficial characteristics to the wearable sensor system. By way of example, the wearable sensor device is typically retained over a wearer's skin through the attachment of micro-probes along with adhesives (e.g., tape) applied around the micro-probes. The unpredictable surface condition of the wearer (e.g., perspiration, rash desquamation, allergic reaction, etc.) often causes accelerated degradation of the adhesives of the interface module. It is observed that the power unit (e.g., battery) of the functional module (e.g., module 10) often out-lasts the operational duration of the adhesive layer of the interface module (e.g., module 20). As it would be wasteful to replace an otherwise functional active sensor merely due to worn-out adhesives, the separable modular design of the instant disclosure allows economical replacement of only the user interface portion (e.g., module 20) while retaining the yet-operational active function components (e.g., module 10).

In some embodiments, the conductive posts 1022 of the function module 10 are provided to electrically couple the plurality of electrodes 202 and the switch plate 203. In some embodiments, the switch plate 203 may comprise a conductive plate configured to electrically couple at least two of the conductive posts 1022, whereby the detection of proper connection between the conductive post 1022 (of the function module 10) and the switch plate 203 (of the interface module 20) may allow the wearable device to initiate operation of the function module 10.

In some embodiments, the top portion 101 of the function module 10 further includes a fastening mechanism 1011. In some embodiments, the carrier 201 of the user interface module 20 may be correspondingly provided with a fastening member 2011. The fastening mechanism 1011 of the function module 10 and the fastening member 2011 of the user interface module 20 may detachably join each other to form a composite device enclosure. By way of example, the fastening mechanism 1011 of the function module 10 and the fastening member 2011 of the user interface module 20 may be a pair of male/female latching members configured to structurally engage each other.

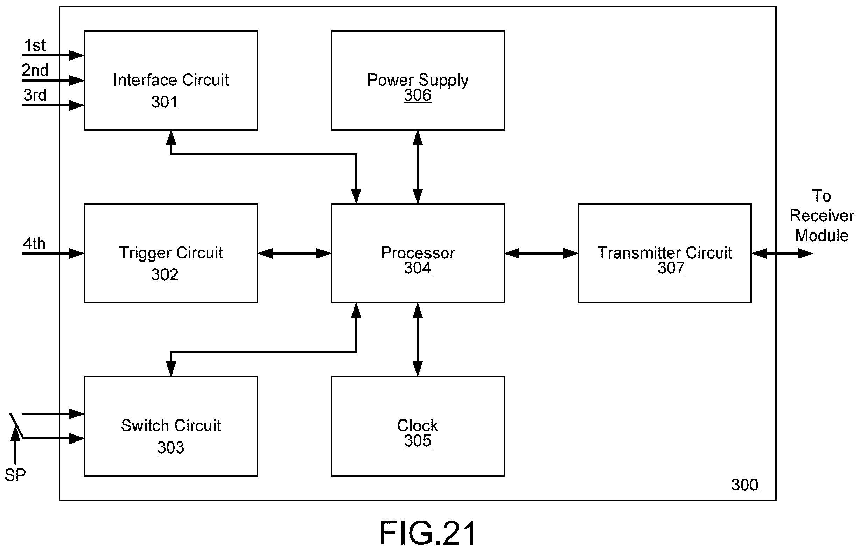

Referring ahead to FIG.21, which illustrates a system modular block diagram of a functional module for a wearable sensor device in accordance with some embodiments of the present disclosure. As discussed previously, the incorporation of shorter micro-probes potentially increases probe retention challenge. For portable devices that run on on-board battery, conservation of battery power would be desirable for prolonging operational duration of the wearable sensor device. To that end, embodiments in accordance with the instant disclosure provide a trigger mechanism capable of detecting device displacement/misplacement of the wearable device. The trigger mechanism may further be configured to regulate power consumption of the wearable device upon determination of sensor misplacement, thereby enabling greater operational efficacy of the wearable sensor device.

In the illustrated embodiment, the function module 300 includes a power supply 306, an interface circuit 301, a transmitter circuit 307, a switch circuit 303, and a processor 304. In some embodiments, the function module 300 further includes a trigger circuit 302. In some embodiments, the function module 300 further includes a clock 305. The interface circuit 301, the transmitter circuit 307, and the switch circuit 303 are operationally coupled to the processor 304. In some embodiments, the power supply 306 (such as a battery) is sealingly arranged in the housing of the function module 300 to provide power for the user interface module 300 in a substantially waterproof manner.

In some embodiments, interface circuit 301 is arranged in signal communication with (e.g., detachably coupled to) a working electrode (whose detected signal is represented by the arrow labeled "1^{st}"), a reference electrode (labeled "2^{nd}"), and a counter electrode (labeled "3^{rd}"). The interface circuit 301 is configured to translate biometrical signals (e.g., signal from biochemical reactions of a device wearer) detected/collected by the electrodes to a measurable value (such as electrical voltage, current, or resistance).

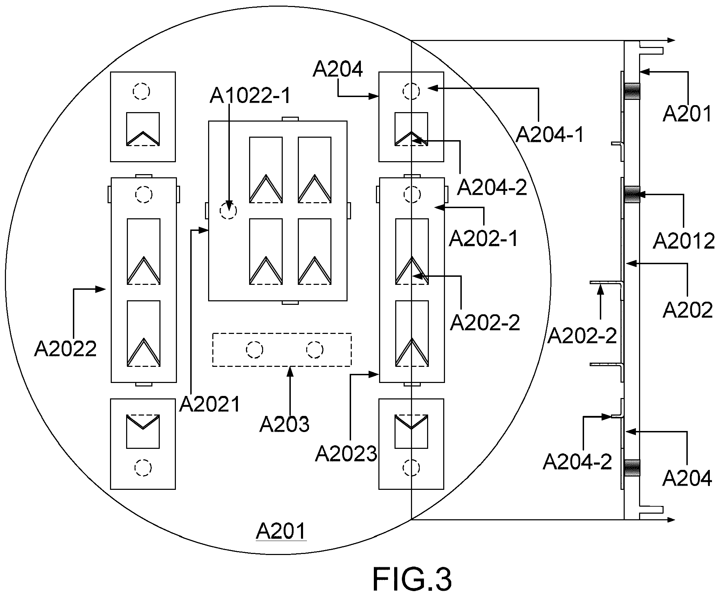

According to the invention, trigger circuit 302 is arranged in signal communication with (e.g., detachably coupled to) a trigger electrode (labeled "4^{th}"). The trigger circuit 302 is configured to determine whether the user interface module is correctly attached to the device wearer. The trigger circuit 302 is operatively associated with one or more trigger electrode (e.g., electrode A204 as shown in FIG.3) in the user interface module. Based on the detected signal from the trigger electrode, the trigger circuit 302 determines whether the wearable device is properly retained on a wearer's skin. In some embodiments, the trigger circuit 302 is configured to cease function of other functional components (e.g., turning off) upon detection of improper placement of the wearable device on a wear's skin, so as to save battery life until placement of the wearable sensor device is rectified. In some embodiments, the trigger circuit 302 may be configured to generate a warning signal to alert a user of the misplacement of the sensor device.

In an exemplary embodiment, the trigger circuit 302 may incorporate a comparator having a contact signal from the trigger electrode (labeled "4^{th}") as an input. The contact signal may be an electrical signal such as resistance, capacitance, or inductance. The trigger circuit 302 compares the contact signal to a signal of a predetermined level and generates the output signal. The output signal may then be used as a basis for activating other functional components in the functional module as previously discussed.

In some embodiments, switch circuit 303 is arranged in signal connection with (e.g., detachably coupled to) the switch plate SP (e.g., element 203 as shown in the example of FIG. 1). The switch circuit 303 is configured to determine whether the user interface module (e.g., module 20 as shown in FIG. 1) is in proper signal connection with the function module (e.g., module 10 as shown in FIG. 1).

In the illustrated embodiment, clock 305 is coupled to the processor 304 and configured to synchronize the circuits in the user interface module 300. In some embodiments, processor 304 is configured to receive signal(s) from the interface circuit 301, the trigger circuit 302, and the switch circuit 303, and further convert those signals into an output signal for transmission by the transmitter circuit 307 to an external device (such as a receiver module of an off-board device). In some embodiments, the transmitter circuit 307 is configured to wirelessly transmit signal (such as Bluetooth signal) to an off-board receiver device.

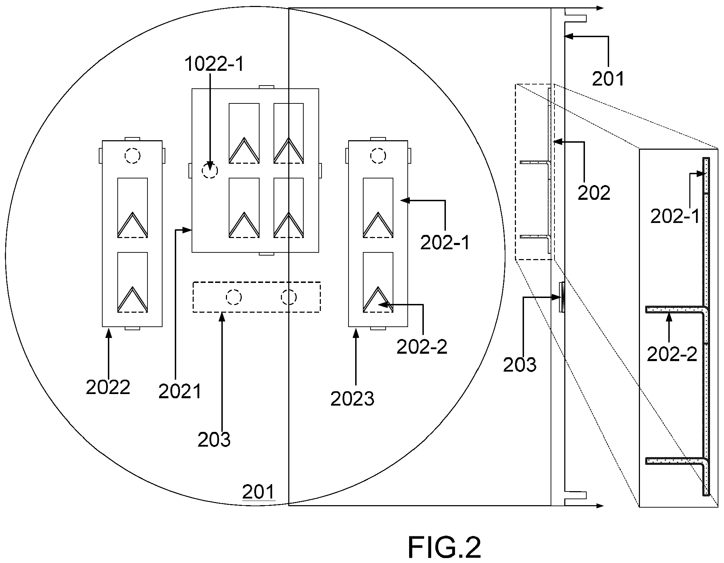

Referring back to FIG.2, which illustrates a planar view of a user interface module of a wearable sensing device according to some embodiments of the present disclosure. In addition, a cross-sectional view of the use interface module (e.g., alone a cutline through electrode 202W) is further provided on the right side of the overhead illustration. In some embodiments, a first group of microprobes 202W is arranged protruding from a contact face of the carrier 201. The first group of microprobes 202W may be associated with a first functional electrode of the wearable sensing device (e.g., correspondingly coupled to a working electrode (W)). In some embodiments, a second group of microprobes 202R is protrudingly arranged over the contact face of the carrier 201. Function wise, the second group of microprobes 202R may be associated with a second functional electrode of the wearable sensing device (e.g., correspond to a reference electrode (R)). In some embodiments, a third group of microprobes 202C is protruding from the contact face of the carrier 201. The third group of microprobes 202C may be associated with a third functional electrode of the wearable sensing device (e.g., correspond to a counter electrode (C)). Nevertheless, the exemplary association of the second group of microprobes 202R and the third group of microprobes 202C to the reference electrode (R) and the counter electrode (C) are arbitrarily illustrative, and may be interchangeable with each other.

The first, second, and third group of microprobes respectively comprise piercers 202-2 that extend from a perforated plate 202-1. In some embodiments, a bottom face of the perforated plate 202-1 defines the contact face of the carrier 201 (i.e.., an inner/bottom surface of the wearable device that comes into contact with the skin of a device wearer). In some embodiments, the height of the microprobes refers to a height of the piercers 202-2 (i.e., a normal distance from the tip of a piercer 202-2 to the contact face of the carrier 201).

The probe of the first group of microprobes 202W defines a first height. Likewise, the probe from the second group of microprobes 202R defines a second height. The probe from the third group of microprobes 202C has a third height. In some embodiments, the first height, the second height and the third height are substantially identical. In some embodiments, the first height, the second height, and the third height are less than 2000um. In some embodiments, the first height, the second height, and the third height are about 1000um. Since an average thickness of an epidermis is 100um and an average thickness of a dermis is 2000um, the first height, the second height, and the third height may range may be greater than 100um but less than 2000um.

In some embodiments, a coupling region 1022-1 is provided on the perforated plates (e.g., plate 202-1). In the illustrated embodiment, the coupling region(s) (e.g., region 1022-1) of the perforated plates are exposed through the back face of the carrier 201 (e.g., as indicated by the dashed lines). In some embodiments, conductive posts of the function module (e.g., module 10 has shown in FIG.1) are correspondingly arranged to establish electrical coupling to the user interface module through the coupling region 1022-1 of the perforated plates 202-1, where the user interface module may detachably (and signal communicatively) join the function module.

FIG.3 illustrates a planar view of a user interface module of a wearable sensing device according to some embodiments of the present disclosure. In addition, a cross-sectional view (e.g., along a cutline through electrodes A204 and A202C) is also provided in supplement to the overhead illustration.

The user interface module includes a carrier A201, a plurality of biometric sensing electrodes (e.g., A202W/R/C, each comprises an array of probes/piercers A202-2) disposed on a contact face of the carrier A201, and a switch plate A203 disposed on a back face of the carrier A201. As shown in the instant embodiment, the conductive posts (e.g., at location indicated by label A2012) of the function module (e.g., module 10 as shown in FIG.1) may respectively establish electrical connection with the plurality of electrodes A202 and the switch plate A203. In some embodiments, the switch plate A203 comprises a conductive plate configured to electrically couple at least two conductive posts. Upon proper connection between the switch plate A203 and the conductive posts (e.g., through hole A2012), the function module (e.g., module 10) and the interface module (e.g., module 20) may be signal communicatively connected, and the functionality of the wearable sensor device may be initiated.

In typical embodiments, the electrodes A202 include a working electrode A202W, a reference electrode A202R, and a counter electrode A202C. In the illustrated embodiment, the working electrode A202R corresponds to a first group of microprobes (e.g., as shown by the group of 4 protruding piercers on the electrode plate A202W). Likewise, the reference electrode A202R corresponds to a second group of microprobes (e.g., as illustrated by the group of 2 protruding piercers on electrode plate A202R arranged on the left hand side of the working electrode A202W). Similarly, the counter electrode A202C corresponds to a third group of microprobes (e.g., the group of 2 probe piercers on electrode plate A202C on the right side of the working electrode A202W).

In the illustrated embodiment, the user interface module further includes a trigger electrode A204. In some embodiments, the trigger electrode corresponds to a fourth group of microprobe (e.g., which comprises 4 separately distributed probe piercers arranged around the four corners of the working electrode A202W). The trigger electrode A204 is associated with a trigger circuit (e.g., circuit 302 shown in FIG.21) in the function module, and is configured to generate an alert signal when sensing a drop of contact signal below the predetermined signal level.

In some embodiments, the first, second, and third groups of microprobes respectively comprise an array of piercers A202-2 extend from a perforated plate (e.g., plate A202-1). On the other hand, the fourth group of microprobe A204 may include one or more protruding piercer(s) A204-2.

In some embodiments, the height of the microprobes refers to the height of the piercers A202-2, A204-2. A probe from the first group of microprobes A202W generally defines a first height. A probe from the second group of microprobes A202R has a second height. A probe from the third group of microprobes A202C have a third height. A probe from the fourth group of microprobe A204 has a fourth height. In some embodiments, the first height and the third height are substantially identical. In some embodiments, the second height is substantially the same with the first height and the third height. In some embodiments, the fourth height is less than the first height and the third height. In some embodiments, the fourth height is less than the second height. In the illustrated embodiment, the longer probes A202-2 of probe groups A202W, A202R, and A202C correspond respectively to the working, the reference, and the counter electrodes, while the shorter probes A204-2 from the fourth group of microprobes A204 corresponds to the trigger electrode (e.g., associated with a trigger circuit 302 as shown in FIG.21).

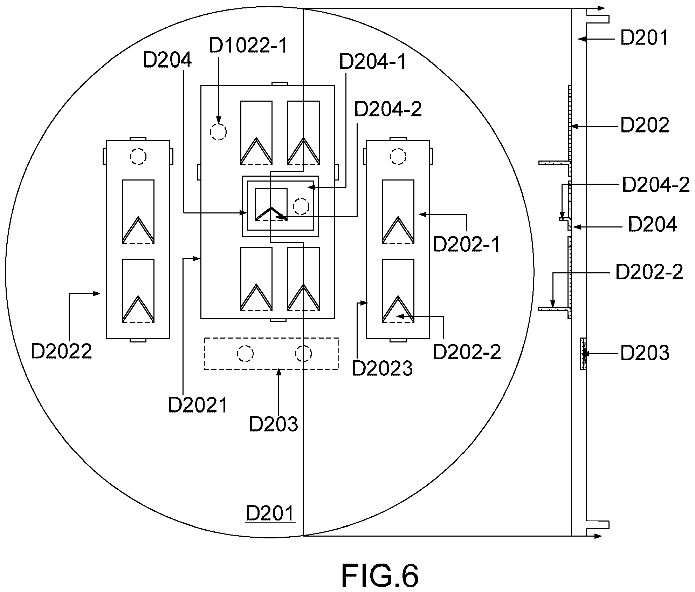

Depending on layout arrangement of the microprobe/piercer, the fourth group of microprobe may be of a single piercer configuration (e.g., as illustrated in FIG.6) or of a multi-piercer setup. For instance, the embodiment of FIG.3 incorporates a multi-piercer setup, in which 4 microprobes A204-2 in association with the trigger electrode A204 are arranged around the four corners of the working electrode A202W.

In some embodiments, the first height and the second height are less than 2000um. In some embodiments, the first height and the second height are substantially 1000um. In some embodiments, the fourth height is less than 100um. In some embodiments, a ratio between the fourth height and the first height is 1.8:20. In some other embodiments, a ratio between the fourth height and the first height is 2.0: 18.5. In some other embodiments, a ratio between the fourth height and the first height is 2.2: 18.0. The shorter length of the trigger electrode probes A204-2 allows the group of trigger probes to serve a forerunner for the rest of the functional electrode probes. By way of example, by strategically placing the shorter trigger probe(s) A204-2 around the working electrode A202W, the retention status of the shorter probes may serve as inherent indication for the longer piercers of the functional electrodes. For instance, if the shorter trigger probes A204-2 receive adequate signal strength that indicates proper retention thereof over a wearer's skin, it would naturally imply the proper seating status of the longer micro-piercers (e.g., A202-2).

In some embodiments, the ratio between the number of microprobes in the first group of microprobes A202W and the number of microprobes in the third group of microprobes A202C ranges between 2:1 to 3:1. In some embodiments, the number of microprobes in the first group of microprobes A202W is four, and the number of microprobes in the third group of microprobes A202C is two. In some embodiments, the number of microprobes in the first group of microprobes A202W is nine, and the number of microprobes in the third group of microprobes A202C is three. In some embodiments, the number of microprobe of the fourth group of microprobe A204 is one. In some other embodiments, the number of microprobe of the fourth group of microprobe A204 is greater than one.

In some embodiments, a coupling region A1022-1 of the perforated plates A202-1, A204-1 of the first, second, third, and fourth groups of microprobes are exposed through the back face of the carrier A201. In some embodiments, conductive posts of the function module are correspondingly electrically coupled to the coupling region A1022-1 of the perforated plates A202-1 when the user interface module is detachably joined to the function module.

In some embodiments, the first group of microprobes A202W is disposed between the second group of microprobes A202R and the third group of microprobes A202C. In some embodiments, the fourth group of microprobe A204 is arranged projectively offset from the perforated plate A202-1 of the first group of microprobe A202W. In some embodiments, the fourth group of microprobe A204 is arranged at a periphery of the contact face and around the first group of microprobes A202W. As shown in FIG.3, the fourth group of microprobe A204 is arranged in at least one corner of the perforated plate A202-1 of the first group of microprobe A202W. In some other embodiments, the fourth group of microprobe A204 is arranged in four corners of the perforated plate A202-1 of the first group of microprobe A202W.

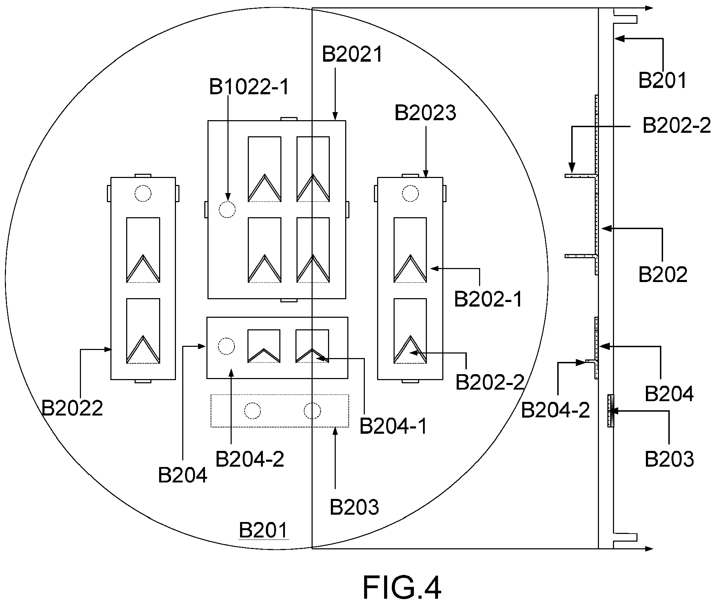

FIG.4 illustrates a planar view of a user interface module of a wearable sensing device according to some embodiments of the present disclosure. In addition, a cross-sectional view (e.g., along a cutline through electrodes B204 and B202W) is also provided in supplement to the overhead illustration.

The user interface module includes a carrier B201, a plurality of biometric sensing electrodes (e.g., B202W/R/C, each comprises an array of probes/piercers B202-2) disposed on a contact face of the carrier B201, and a switch plate B203 disposed on a back face of the carrier B201. As shown in the instant embodiment, the conductive posts of the function module (e.g., module 10 as shown in FIG.1) may respectively establish electrical connection with the plurality of electrodes B202 and the switch plate B203. In some embodiments, the switch plate B203 is a conductive plate configured to electrically couple at least two conductive posts. Upon proper connection between the switch plate B203 and the conductive posts, the function module (e.g., module 10) and the interface module (e.g., module 20) may be signal communicatively connected, and the functionality of the wearable sensor device may be initiated.

In typical embodiments, the electrodes B202 include a working electrode B202W, a reference electrode B202R, and a counter electrode B202C. In the illustrated embodiment, the working electrode B202R corresponds to a first group of microprobes (e.g., as shown by the group of 4 protruding piercers on the electrode plate B202W). Likewise, the reference electrode B202R corresponds to a second group of microprobes (e.g., as illustrated by the group of 2 protruding piercers on electrode plate B202R arranged on the left hand side of the working electrode B202W). Similarly, the counter electrode B202C corresponds to a third group of microprobes (e.g., the group of 2 probe piercers on electrode plate B202C on the right side of the working electrode B202W).

In the illustrated embodiment, the user interface module further includes a trigger electrode B204. In some embodiments, the trigger electrode corresponds to a fourth group of microprobe (e.g., which comprises 2 probe piercers arranged in the vicinity of the working electrode B202W). The trigger electrode B204 is associated with a trigger circuit (e.g., circuit 302 shown in FIG.21) in the function module configured to generate an alert signal when sensing a drop of contact signal below the predetermined signal level.

In some embodiments, the first, second, and third groups of microprobes respectively comprise an array of piercers B202-2 extend from a perforated plate (e.g., plate B202-1). On the other hand, the fourth group of microprobe B204 may include one or more protruding piercer(s) B204-2.

In some embodiments, the height of the microprobes refers to the height of the piercers B202-2, B204-2. A probe from the first group of microprobes B202W generally defines a first height. A probe from the second group of microprobes B202R has a second height. A probe from the third group of microprobes B202C has a third height. The fourth group of microprobe B204 has a fourth height. In some embodiments, the first height and the third height are substantially identical. In some embodiments, the second height is substantially the same with the first height and the third height. In some embodiments, the fourth height is less than the first height and the third height. In some embodiments, the fourth height is less than the second height. In the illustrated embodiment, the longer probes B202-2 of probe group B202W, B202R, and B202C correspond respectively to the working, the reference, and the counter electrodes, while the shorter probes B204-2 from the fourth group of microprobes B204 corresponds to the trigger electrode (e.g., associated with a trigger circuit 302 as shown in FIG.21).

Depending on layout arrangement of the microprobe/piercer, the fourth group of microprobe may be of a single piercer configuration (e.g., as illustrated in FIG.6) or of a multi-piercer setup. For instance, the embodiment of FIG.4 incorporates a multi-piercer setup, in which 2 microprobes B204-2 in association with the trigger electrode B204 are arranged around the working electrode B202W.

In some embodiments, the first height and the second height are less than 2000um. In some embodiments, the first height and the second height are substantially 1000um. In some embodiments, the fourth height is less than 100um. In some embodiments, a ratio between the fourth height and the first height is 1.8:20. In some other embodiments, a ratio between the fourth height and the first height is 2.0: 18.5. In some other embodiments, a ratio between the fourth height and the first height is 2.2: 18.0. The shorter length of the trigger electrode probes B204-2 allows the group of trigger probes to serve a forerunner for the rest of the functional electrode probes. By way of example, by strategically placing the shorter trigger probe(s) B204-2 around the working electrode B202W, the retention status of the shorter probes may serve as inherent indication for the longer piercers of the functional electrodes. For instance, if the shorter trigger probes B204-2 receive adequate signal strength that indicates proper retention thereof over a wearer's skin, it would naturally imply the proper seating status of the longer micro-piercers (e.g., B202-2).

In some embodiments, the ratio between the number of microprobes in the first group of microprobes B202W and the number of microprobes in the third group of microprobes B202C ranges between 2:1 to 3:1. In some embodiments, the number of microprobes in the first group of microprobes B202W is four and the number of microprobes in the third group of microprobes B202C is two. In some embodiments, the number of microprobes in the first group of microprobes B202W is nine and the number of microprobes in the third group of microprobes B202C is three. In some embodiments, the number of microprobe of the fourth group of microprobe B204 is one. In some other embodiments, the number of microprobe of the fourth group of microprobe B204 is greater than one.

In some embodiments, a coupling region B1022-1 of the perforated plates B202-1, B204-1 of the first, second, third, and fourth group of microprobes are exposed through the back face of the carrier B201. In some embodiments, conductive posts of the function module are correspondingly electrically coupled to the coupling region B 1022-1 of the perforated plates B202-1 when the user interface module is detachably joined to the function module.

In some embodiments, the first group of microprobes B202W is disposed between the second group of microprobes B202R and the third group of microprobes B202C. In some embodiments, the fourth group of microprobe B204 is arranged projectively offset the perforated plate B202-1 of the first group of microprobe B202W. In some embodiments, the fourth group of microprobe B204 is arranged laterally proximate the first group of microprobe B202W. As shown in FIG.4, the fourth group of microprobe B204 is arranged along a side of the perforated plate B202-1 of the first group of microprobe B202W. In some embodiments, the fourth group of microprobe B204 is disposed between the second group of microprobes B202R and the third group of microprobes B202C.In some embodiments, a distance between the perforated plate B204-1 of the fourth group of microprobe B204 and the perforated plate B202-1 of the first group of microprobe B202W ranges between 100um to 2000um.

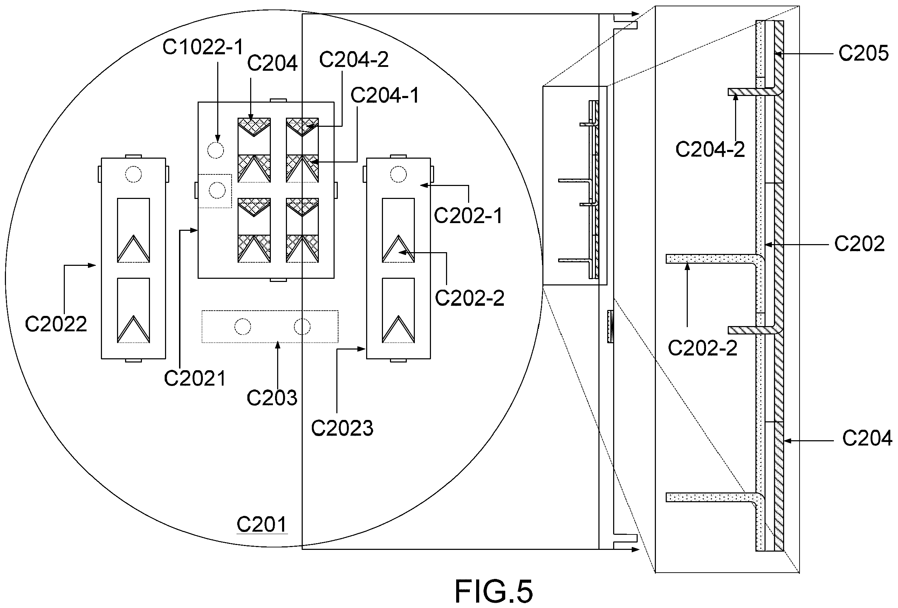

FIG.5 illustrates a planar view of a user interface module of a wearable sensing device according to some embodiments of the present disclosure. In addition, a cross-sectional view (e.g., along a cutline through electrodes C204 and C202W) is also provided in supplement to the overhead illustration.

The user interface module includes a carrier C201, a plurality of biometric sensing electrodes (e.g., C202W/R/C, each comprises an array of probes/piercers C202-2) disposed on a contact face of the carrier C201, and a switch plate C203 disposed on a back face of the carrier C201. As shown in the instant embodiment, the conductive posts of the function module (e.g., module 10 as shown in FIG.1) may respectively establish electrical connection with the plurality of electrodes C202 and the switch plate C203. In some embodiments, the switch plate C203 is a conductive plate configured to electrically couple at least two conductive posts. Upon proper connection between the switch plate C203 and the conductive posts, the function module (e.g., module 10) and the interface module (e.g., module 20) may be signal communicatively connected, and the functionality of the wearable sensor device may be initiated.

In typical embodiments, the electrodes C202 include a working electrode C202W, a reference electrode C202R, and a counter electrode C202C. In the illustrated embodiment, the working electrode C202R corresponds to a first group of microprobes (e.g., as shown by the group of 4 protruding piercers on the electrode plate C202W). Likewise, the reference electrode C202R corresponds to a second group of microprobes (e.g., as illustrated by the group of 2 protruding piercers on electrode plate C202R arranged on the left hand side of the working electrode C202W). Similarly, the counter electrode C202C corresponds to a third group of microprobes (e.g., the group of 2 probe piercers on electrode plate C202C on the right side of the working electrode C202W).

In the illustrated embodiment, the user interface module further includes a trigger electrode C204. In some embodiments, the trigger electrode corresponds to a fourth group of microprobe (e.g., which comprises 4 probe piercers overlapping the working electrode C202W). The trigger electrode C204 is associated with a trigger circuit (e.g., circuit 302 shown in FIG.21) in the function module configured to generate an alert signal when sensing a drop of contact signal below the predetermined signal level.

In some embodiments, the first, second, and third groups of microprobes respectively comprise an array of piercers C202-2 extending from a perforated plate (e.g., plate C202-1). On the other hand, the fourth group of microprobe C204 may include one or more protruding piercer(s) C204-2.

In some embodiments, the first group of microprobes C202W is disposed between the second group of microprobes C202R and the third group of microprobes C202C. In some embodiments, the fourth group of microprobe C204 is arranged projectively overlap the perforated plate C202-1 of the first group of microprobe C202W. In some embodiments, the perforated plate C202-1 of the first group of microprobe C202W is disposed above the perforated plate C204-1 of the fourth group of microprobe C204 as shown in FIG.5. In some other embodiments, the perforated plate C202-1 of the first group of microprobe C202W is disposed below the perforated plate C204-1 of the fourth group of microprobe C204. In some embodiments, an insulator C205 is disposed between the perforated plate C202-1 of the first group of microprobe C202W and the perforated plate C204-1 of the fourth group of microprobe C204 to prevent cross talk. In some embodiments, a thickness of the insulator C205 ranges between 2um to 80um. In some embodiments, the through holes of the first group of microprobes C202W, the through holes of the fourth group of microprobe C204, and through holes of the insulator C205 may be aligned with each other. In some embodiments, the through holes of the first group of microprobes C202W has an area greater than that of the through holes of the fourth group of microprobe C204. In this way, a portion of the perforated plate C204-1 is exposed from the through holes of the first group of microprobes C202W.

In some embodiments, the height of the microprobes refers to the height of the piercers C202-2, C204-2. A probe from the first group of microprobes C202W have a first height. A probe from the second group of microprobes C202R has a second height. A probe from the third group of microprobes C202C has a third height. A probe from the fourth group of microprobe C204 has a fourth height. In the exemplary embodiment shown in FIG.5, the fourth height may be the height of the piercer C204-2 protruding from the surface of the perforated plate C202-1 of the first group of microprobe C202W. In some embodiments, the first height and the third height are substantially identical. In some embodiments, the second height is substantially the same with the first height and the third height. In some embodiments, the fourth height is less than the first height and the third height. In some embodiments, the fourth height is less than the second height. In the illustrated embodiment, the longer probes C202-2 of probe group C202W, C202R, and C202C correspond respectively to the working, the reference, and the counter electrodes, while the shorter probes C204-2 from the fourth group of microprobes C204 corresponds to the trigger electrode (e.g., associated with a trigger circuit 302 as shown in FIG.21).

Depending on layout arrangement of the microprobe/piercer, the fourth group of microprobe may be of a single piercer configuration (e.g., as illustrated in FIG.6) or of a multi-piercer setup. For instance, the embodiment of FIG.5 incorporates a multi-piercer setup, in which 4 microprobes C204-2 in association with the trigger electrode C204 are arranged to protrude from through holes of the working electrode C202W.

In some embodiments, the first height and the third height are less than 2000um. In some embodiments, the first height and the third height are substantially 1000um. In some embodiments, the fourth height is less than 100um. In some embodiments, a ratio between the fourth height and the first height is 1.8:20. In some other embodiments, a ratio between the fourth height and the first height is 2.0: 18.5. In some other embodiments, a ratio between the fourth height and the first height is 2.2: 18.0. The shorter length of the trigger electrode probes C204-2 allows the group of trigger probes to serve a forerunner for the rest of the functional electrode probes. By way of example, by strategically placing the shorter trigger probe(s) C204-2 over the working electrode C202W, the retention status of the shorter probes may serve as inherent indication for the longer piercers of the functional electrodes. For instance, if the shorter trigger probes C204-2 receive adequate signal strength that indicates proper retention thereof over a wearer's skin, it would naturally imply the proper seating status of the longer micro-piercers (e.g., C202-2).

In some embodiments, the ratio between the number of microprobes in the first group of microprobes C202W and the number of microprobes in the third group of microprobes C202C ranges between 2:1 to 3:1. In some embodiments, the number of microprobes in the first group of microprobes C202W is four and the number of microprobes in the third group of microprobes C202C is two. In some embodiments, the number of microprobes in the first group of microprobes C202W is nine and the number of microprobes in the third group of microprobes C202C is three. In some embodiments, the number of microprobe of the fourth group of microprobe C204 is the same as the number of microprobes in the first group of microprobes C202W. In some other embodiments, the number of microprobe of the fourth group of microprobe C204 is one. In some other embodiments, the number of microprobe of the fourth group of microprobe C204 is greater than one.

In some embodiments, a coupling region C1022-1 of the perforated plates C202-1, C204-1 of the first, second, third, and fourth group of microprobes are exposed through the back face of the carrier C201. In some embodiments, conductive posts of the function module are correspondingly electrically coupled to the coupling region C1022-1 of the perforated plates C202-1 when the user interface module is detachably joined to the function module.

When the perforated plate C202-1 of the first group of microprobe C202W and the perforated plate C204-1 of the fourth group of microprobe C204 overlap each other, the perforated plate disposed closest to the back face of the carrier C201 is formed to have a notch. In this way, a corresponding conductive post of the function module will be able to electrically couple to the perforated plate disposed farthest from the back face of the carrier C201. In the exemplary embodiment shown in FIG.5, the notch is formed on the perforated plate C204-1 of the fourth group of microprobe C204 since the perforated plate C204-1 is disposed closes to the carrier C201.

FIG.6 illustrates a planar view of a user interface module of a wearable sensing device according to some embodiments of the present disclosure. In addition, a cross-sectional view (e.g., along a cutline through electrodes D204 and D202W) is also provided in supplement to the overhead illustration.

The user interface module includes a carrier D201, a plurality of biometric sensing electrodes (e.g., D202W/R/C, each comprises an array of probes/piercers D202-2) disposed on a contact face of the carrier D201, and a switch plate D203 disposed on a back face of the carrier D201. As shown in the instant embodiment, the conductive posts of the function module (e.g., module 10 as shown in FIG.1) may respectively establish electrical connection with the plurality of electrodes D202 and the switch plate D203. In some embodiments, the switch plate D203 is a conductive plate configured to electrically couple at least two conductive posts. Upon proper connection between the switch plate D203 and the conductive posts, the function module (e.g., module 10) and the interface module (e.g., module 20) may be signal communicatively connected, and the functionality of the wearable sensor device may be initiated.

In typical embodiments, the electrodes D202 include a working electrode D202W, a reference electrode D202R, and a counter electrode D202C. In the illustrated embodiment, the working electrode D202R corresponds to a first group of microprobes (e.g., as shown by the group of 4 protruding piercers on the electrode plate D202W). Likewise, the reference electrode D202R corresponds to a second group of microprobes (e.g., as illustrated by the group of 2 protruding piercers on electrode plate D202R arranged on the left hand side of the working electrode D202W). Similarly, the counter electrode D202C corresponds to a third group of microprobes (e.g., the group of 2 probe piercers on electrode plate D202C on the right side of the working electrode D202W).

In the illustrated embodiment, the user interface module further includes a trigger electrode D204. In some embodiments, the trigger electrode corresponds to a fourth group of microprobe (e.g., which comprises 1 probe piercer arranged at the central area of the working electrode D202W). The trigger electrode D204 is associated with a trigger circuit (e.g., circuit 302 shown in FIG.21) in the function module configured to generate an alert signal when sensing a drop of contact signal below the predetermined signal level.

In some embodiments, the first, second, and third groups of microprobes respectively comprise an array of piercers D202-2 extend from a perforated plate (e.g., plate D202-1). On the other hand, the fourth group of microprobe D204 may include one or more protruding piercer(s) D204-2.

In some embodiments, the height of the microprobes refers to the height of the piercers D202-2, D204-2. A probe from the first group of microprobes D202W has a first height. A probe from the second group of microprobes D202R has a second height. A probe from the third group of microprobes D202C has a third height. A probe from the fourth group of microprobe D204 has a fourth height. In some embodiments, the first height and the third height are substantially identical. In some embodiments, the second height is substantially the same with the first height and the third height. In some embodiments, the fourth height is less than the first height and the third height. In some embodiments, the fourth height is less than the second height. In the illustrated embodiment, the longer probes D202-2 of probe group D202W, D202R, and D202C correspond respectively to the working, the reference, and the counter electrodes, while the shorter probes D204-2 from the fourth group of microprobes D204 corresponds to the trigger electrode (e.g., associated with a trigger circuit 302 as shown in FIG.21).

Depending on layout arrangement of the microprobe/piercer, the fourth group of microprobe may be of a single piercer configuration or of a multi-piercer setup. For instance, the embodiment of FIG.6 incorporates a single piercer setup, in which 1 microprobe D204-2 in association with the trigger electrode D204 is arranged at the central area of the working electrode D202W.

In some embodiments, the first height and the second height are less than 2000um. In some embodiments, the first height and the second height are substantially 1000um. In some embodiments, the fourth height is less than 100um. In some embodiments, a ratio between the fourth height and the first height is 1.8:20. In some other embodiments, a ratio between the fourth height and the first height is 2.0: 18.5. In some other embodiments, a ratio between the fourth height and the first height is 2.2:18.0. The shorter length of the trigger electrode probes D204-2 allows the group of trigger probes to serve a forerunner for the rest of the functional electrode probes. By way of example, by strategically placing the shorter trigger probe(s) D204-2 around the working electrode D202W, the retention status of the shorter probes may serve as inherent indication for the longer piercers of the functional electrodes. For instance, if the shorter trigger probes D204-2 receive adequate signal strength that indicates proper retention thereof over a wearer's skin, it would naturally imply the proper seating status of the longer micro-piercers (e.g., D202-2).

In some embodiments, the ratio between the number of microprobes in the first group of microprobes D202W and the number of microprobes in the third group of microprobes D202C ranges between 2:1 to 3:1. In some embodiments, the number of microprobes in the first group of microprobes D202W is four and the number of microprobes in the third group of microprobes D202C is two. In some embodiments, the number of microprobes in the first group of microprobes D202W is nine and the number of microprobes in the third group of microprobes D202C is three. In some embodiments, the number of microprobe of the fourth group of microprobe D204 is one. In some other embodiments, the number of microprobe of the fourth group of microprobe D204 is greater than one.

In some embodiments, a coupling region D1022-1 of the perforated plates D202-1, D204-1 of the first, second, third, and fourth group of microprobes are exposed through the back face of the carrier D201. In some embodiments, conductive posts of the function module are correspondingly electrically coupled to the coupling region D1022-1 of the perforated plates D202-1 when the user interface module is detachably joined to the function module.

In some embodiments, the first group of microprobes D202W is disposed between the second group of microprobes D202R and the third group of microprobes D202C. In some embodiments, the fourth group of microprobe D204 is arranged projectively offset the perforated plate D202-1 of the first group of microprobe D202W. In some embodiments, the perforated plate D202-1 of the first group of microprobes D202W is formed to have a hole at a central area to accommodate the fourth group of microprobe D204. In some embodiments, the periphery of the perforated plate D204-1 of the fourth group of microprobe D204 is at a distance from the perforated plate D202-1 of the first group of microprobe D202W to prevent cross talk. In some embodiments, a distance between the perforated plate D204-1 of the fourth group of microprobe D204 and the perforated plate D202-1 of the first group of microprobe D202W ranges between 100um to 2000um.

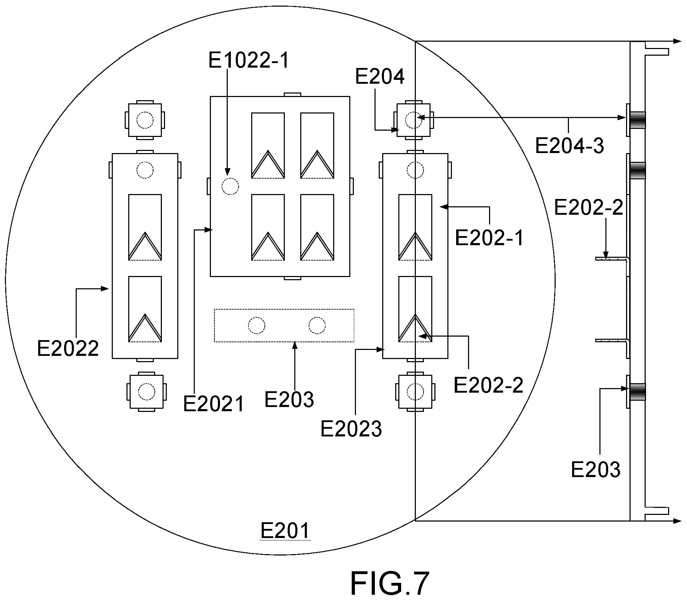

FIG.7 illustrates a planar view of a user interface module of a wearable sensing device according to some embodiments of the present disclosure. In addition, a cross-sectional view (e.g., along a cutline through electrodes E204 and E202C) is also provided in supplement to the overhead illustration.

The user interface module includes a carrier E201, a plurality of biometric sensing electrodes (e.g., E202W/R/C, each comprises an array of probes/piercers E202-2) disposed on a contact face of the carrier E201, and a switch plate E203 disposed on a back face of the carrier E201. As shown in the instant embodiment, the conductive posts of the function module (e.g., module 10 as shown in FIG.1) may respectively establish electrical connection with the plurality of electrodes E202 and the switch plate E203. In some embodiments, the switch plate E203 is a conductive plate configured to electrically couple at least two conductive posts. Upon proper connection between the switch plate E203 and the conductive posts, the function module (e.g., module 10) and the interface module (e.g., module 20) may be signal communicatively connected, and the functionality of the wearable sensor device may be initiated.

In typical embodiments, the electrodes E202 include a working electrode E202W, a reference electrode E202R, and a counter electrode E202C. In the illustrated embodiment, the working electrode E202R corresponds to a first group of microprobes (e.g., as shown by the group of 4 protruding piercers on the electrode plate E202W). Likewise, the reference electrode E202R corresponds to a second group of microprobes (e.g., as illustrated by the group of 2 protruding piercers on electrode plate E202R arranged on the left hand side of the working electrode E202W). Similarly, the counter electrode E202C corresponds to a third group of microprobes (e.g., the group of 2 probe piercers on electrode plate E202C on the right side of the working electrode E202W).

In the illustrated embodiment, the user interface module further includes a trigger electrode E204. In some embodiments, the trigger electrode corresponds to a fourth group of microprobe (e.g., which comprises 4 separately distributed probes arranged around the four corners of the working electrode E202W). The trigger electrode E204 is associated with a trigger circuit (e.g., circuit 302 shown in FIG.21) in the function module configured to generate an alert signal when sensing a drop of contact signal below the predetermined signal level.

In some embodiments, the first, second, and third group of microprobes respectively comprise an array of piercers E202-2 extend from a perforated plate (e.g., plate E202-1). In some embodiments, each microprobe includes only one piercer E202-2. In some other embodiment, each microprobe includes a plurality of piercers E202-2. On the other hand, the fourth group of microprobe E204 have no piercers. In some embodiments, the fourth group of microprobe E204 has a sensing surface E204-3 substantially planar to the perforated plate E202-1 of the first group of microprobes E202W. In some other embodiments, the fourth group of microprobe E204 has a sensing surface E204-3 protruding from the perforated plate E202-1 of the first group of microprobes E202W.

In some embodiments, the height of the first, second, and third groups of microprobes refers to the height of the piercers E202-2. A probe from the first group of microprobes E202W has a first height. A probe from the second group of microprobes E202R has a second height. A probe from the third group of microprobes E202C has a third height. In some embodiments, the height of the fourth groups of microprobe refers to the height of sensing surface E204-3 corresponding to the perforated plate E202-1 of the first group of microprobes E202W. A probe from the fourth group of microprobe E204 has a fourth height. In some embodiments, the first height and the third height are substantially identical. In some embodiments, the second height is substantially the same with the first height and the third height. In some embodiments, the fourth height is less than the first height and the third height. In some embodiments, the fourth height is less than the second height. In the illustrated embodiment, the longer probes E202-2 of probe group E202W, E202R, and E202C correspond respectively to the working, the reference, and the counter electrodes, while the shorter probes E204-3 from the fourth group of microprobes E204 corresponds to the trigger electrode (e.g., associated with a trigger circuit 302 as shown in FIG.21).

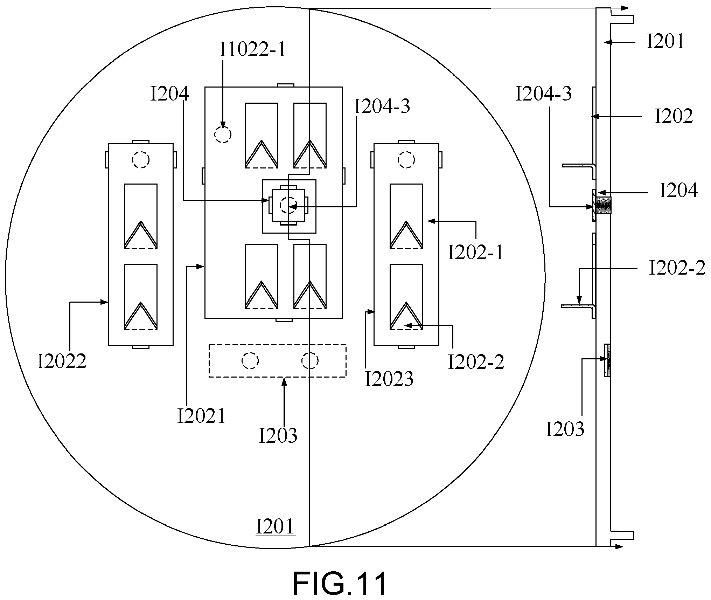

Depending on layout arrangement of the microprobe/piercer, the fourth group of microprobe may be of a single probe configuration (e.g., as illustrated in FIG.11) or of a multi-probes setup. For instance, the embodiment of FIG.7 incorporates a multi-piercer setup, in which 4 microprobes E204-3 in association with the trigger electrode E204 are arranged around the four corners of the working electrode E202W.

In some embodiments, the first height and the second height are less than 2000um. In some embodiments, the first height and the second height are substantially 1000um. The shorter length of the trigger electrode probes E204-3 allows the group of trigger probes to serve a forerunner for the rest of the functional electrode probes. By way of example, by strategically placing the shorter trigger probe(s) E204-3 around the working electrode E202W, the retention status of the shorter probes may serve as inherent indication for the longer piercers of the functional electrodes. For instance, if the shorter trigger probes E204-3 receive adequate signal strength that indicates proper retention thereof over a wearer's skin, it would naturally imply the proper seating status of the longer micro-piercers (e.g., E202-2).

In some embodiments, the ratio between the number of microprobes in the first group of microprobes E202W and the number of microprobes in the third group of microprobes E202C ranges between 2:1 to 3:1. In some embodiments, the number of microprobes in the first group of microprobes E202W is four and the number of microprobes in the third group of microprobes E202C is two. In some embodiments, the number of microprobes in the first group of microprobes E202W is nine and the number of microprobes in the third group of microprobes E202C is three. In some embodiments, the number of microprobe of the fourth group of microprobe E204 is one. In some other embodiments, the number of microprobe of the fourth group of microprobe E204 is greater than one.

In some embodiments, a coupling region E1022-1 of the perforated plates E202-1 of the first, second, and third group of microprobes are exposed through the back face of the carrier E201. In some embodiments, conductive posts of the function module are correspondingly electrically coupled to the coupling region E1022-1 of the perforated plates E202-1 when the user interface module is detachably joined to the function module. In some embodiments, a backside of the sensing surface E204-3 is exposed through the back face of the carrier E201. In some embodiments, a conductive post of the function module is correspondingly electrically coupled to the backside of the sensing surface E204-3. During application, the sensing surface E204-3 may be in contact with the skin of the user without piercing the skin of the user. The conductive post is configured to apply force to the sensing surface E204-3 and prevent the sensing surface E204-3 from retracting from the skin when in use.

In some embodiments, the first group of microprobes E202W is disposed between the second group of microprobes E202R and the third group of microprobes E202C. In some embodiments, the fourth group of microprobe E204 is arranged projectively offset the perforated plate E202-1 of the first group of microprobe E202W. In some embodiments, the fourth group of microprobe E204 is arranged at periphery of the contact face and around the first group of microprobes E202W. As shown in FIG.7, the fourth group of microprobe E204 is arranged in at least one corner of the perforated plate E202-1 of the first group of microprobe E202W. In some other embodiments, the fourth group of microprobe E204 is arranged in four corners of the perforated plate E202-1 of the first group of microprobe E202W.

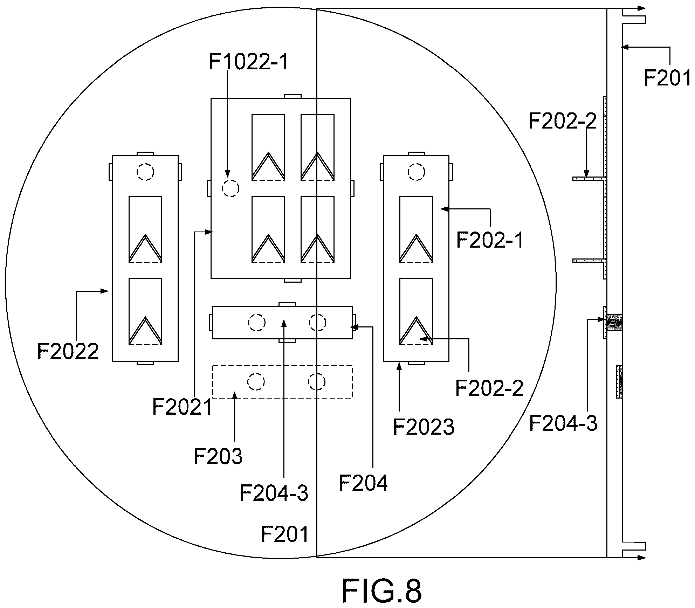

FIG.8 illustrates a planar view of a user interface module of a wearable sensing device according to some embodiments of the present disclosure. In addition, a cross-sectional view (e.g., along a cutline through electrodes F204 and F202W) is also provided in supplement to the overhead illustration.

The user interface module includes a carrier F201, a plurality of biometric sensing electrodes (e.g., F202W/R/C, each comprises an array of probes/piercers F202-2) disposed on a contact face of the carrier F201, and a switch plate F203 disposed on a back face of the carrier F201. As shown in the instant embodiment, the conductive posts of the function module (e.g., module 10 as shown in FIG.1) may respectively establish electrical connection with the plurality of electrodes F202 and the switch plate F203. In some embodiments, the switch plate F203 is a conductive plate configured to electrically couple at least two conductive posts. Upon proper connection between the switch plate F203 and the conductive posts, the function module (e.g., module 10) and the interface module (e.g., module 20) may be signal communicatively connected, and the functionality of the wearable sensor device may be initiated.

In typical embodiments, the electrodes F202 include a working electrode F202W, a reference electrode F202R, and a counter electrode F202C. In the illustrated embodiment, the working electrode F202R corresponds to a first group of microprobes (e.g., as shown by the group of 4 protruding piercers on the electrode plate F202W). Likewise, the reference electrode F202R corresponds to a second group of microprobes (e.g., as illustrated by the group of 2 protruding piercers on electrode plate F202R arranged on the left hand side of the working electrode F202W). Similarly, the counter electrode F202C corresponds to a third group of microprobes (e.g., the group of 2 probe piercers on electrode plate F202C on the right side of the working electrode F202W).

In the illustrated embodiment, the user interface module further includes a trigger electrode F204. In some embodiments, the trigger electrode corresponds to a fourth group of microprobe (e.g., which comprises a probe arranged in the vicinity of the working electrode F202W). The trigger electrode F204 is associated with a trigger circuit (e.g., circuit 302 shown in FIG.21) in the function module configured to generate an alert signal when sensing a drop of contact signal below the predetermined signal level.

In some embodiments, the first, second, and third group of microprobes respectively comprise an array of piercers F202-2 extend from a perforated plate (e.g., plate F202-1). In some embodiments, each microprobe includes only one piercer F202-2. In some other embodiment, each microprobe includes a plurality of piercers F202-2. On the other hand, the fourth group of microprobe F204 have no piercers. In some embodiments, the fourth group of microprobe F204 has a sensing surface F204-3 substantially planar to the perforated plate F202-1 of the first group of microprobes F202W. In some other embodiments, the fourth group of microprobe F204 has a sensing surface F204-3 protruding from the perforated plate F202-1 of the first group of microprobes F202W.

In some embodiments, the height of the first, second, and third group of microprobes refers to the height of the piercers F202-2. A probe from the first group of microprobes F202W has a first height. A probe from the second group of microprobes F202R has a second height. A probe from the third group of microprobes F202C has a third height. A probe from the fourth group of microprobe F204 has a fourth height. In some embodiments, the first height and the third height are substantially identical. In some embodiments, the second height is substantially the same with the first height and the third height. In some embodiments, the fourth height is less than the first height and the third height. In some embodiments, the fourth height is less than the second height. In the illustrated embodiment, the longer probes F202-2 of probe group F202W, F202R, and F202C correspond respectively to the working, the reference, and the counter electrodes, while the shorter probes F204-3 from the fourth group of microprobes F204 corresponds to the trigger electrode (e.g., associated with a trigger circuit 302 as shown in FIG.21).

Depending on layout arrangement of the microprobe/piercer, the fourth group of microprobe may be of a single piercer configuration or of a multi-piercer setup. For instance, the embodiment of FIG.8 incorporates a single probe setup, in which 1 microprobe F204-3 in association with the trigger electrode F204 is arranged at the vicinity of the working electrode F202W.

In some embodiments, the first height and the second height are less than 2000um. In some embodiments, the first height and the second height are substantially 1000um. The shorter length of the trigger electrode probes F204-3 allows the group of trigger probes to serve a forerunner for the rest of the functional electrode probes. By way of example, by strategically placing the shorter trigger probe(s) F204-3 around the working electrode F202W, the retention status of the shorter probes may serve as inherent indication for the longer piercers of the functional electrodes. For instance, if the shorter trigger probes F204-3 receive adequate signal strength that indicates proper retention thereof over a wearer's skin, it would naturally imply the proper seating status of the longer micro-piercers (e.g., F202-2).

In some embodiments, the ratio between the number of microprobes in the first group of microprobes F202W and the number of microprobes in the third group of microprobes F202C ranges between 2:1 to 3:1. In some embodiments, the number of microprobes in the first group of microprobes F202W is four and the number of microprobes in the third group of microprobes F202C is two. In some embodiments, the number of microprobes in the first group of microprobes F202W is nine and the number of microprobes in the third group of microprobes F202C is three. In some embodiments, the number of microprobe of the fourth group of microprobe F204 is one. In some other embodiments, the number of microprobe of the fourth group of microprobe F204 is greater than one.

In some embodiments, a coupling region F1022-1 of the perforated plates F202-1 of the first, second, and third group of microprobes are exposed through the back face of the carrier F201. In some embodiments, conductive posts of the function module are correspondingly electrically coupled to the coupling region F1022-1 of the perforated plates F202-1 when the user interface module is detachably joined to the function module. In some embodiments, a backside of the sensing surface F204-3 is exposed through the back face of the carrier F201. In some embodiments, a conductive post of the function module is correspondingly electrically coupled to the backside of the sensing surface F204-3. During application, the sensing surface F204-3 may be in contact with the skin of the user without piercing the skin of the user. The conductive post is configured to apply force to the sensing surface F204-3 and prevent the sensing surface F204-3 from retracting from the skin when in use.

In some embodiments, the first group of microprobes F202W is disposed between the second group of microprobes F202R and the third group of microprobes F202C. In some embodiments, the fourth group of microprobe F204 is arranged projectively offset the perforated plate F202-1 of the first group of microprobe F202W. In some embodiments, the fourth group of microprobe F204 is arranged laterally proximate the first group of microprobe F202W. In some embodiments, the fourth group of microprobe F204 is disposed between the second group of microprobes F202R and the third group of microprobes F202C. As shown in FIG.8, the fourth group of microprobe F204 is arranged along a side of the perforated plate F202-1 of the first group of microprobe F202W. In some embodiments, a distance between the perforated plate F204-1 of the fourth group of microprobe F204 and the perforated plate F202-1 of the first group of microprobe F202W ranges between 100um to 2000um.

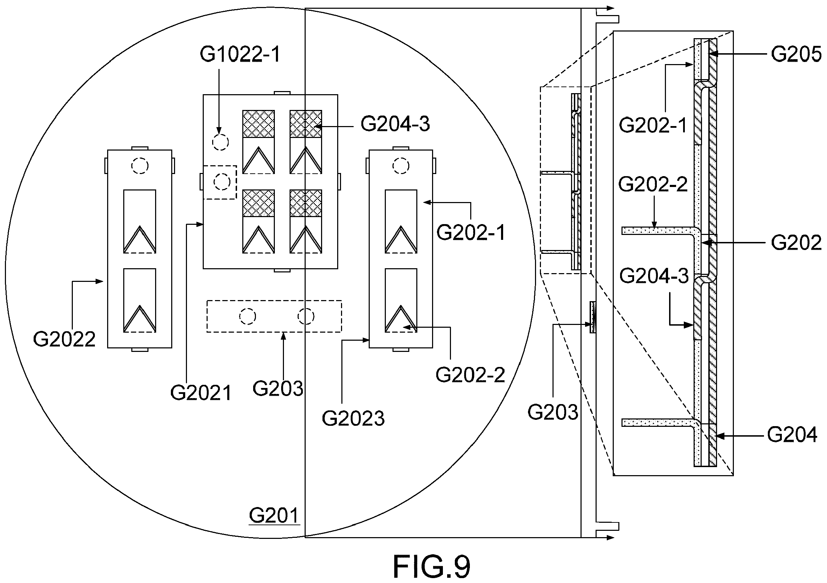

FIG.9 illustrates a planar view of a user interface module of a wearable sensing device according to some embodiments of the present disclosure. In addition, a cross-sectional view (e.g., along a cutline through electrodes G204 and G202W) is also provided in supplement to the overhead illustration.

The user interface module includes a carrier G201, a plurality of biometric sensing electrodes (e.g., G202W/R/C, each comprises an array of probes/piercers G202-2) disposed on a contact face of the carrier G201, and a switch plate G203 disposed on a back face of the carrier G201. As shown in the instant embodiment, the conductive posts of the function module (e.g., module 10 as shown in FIG.1) may respectively establish electrical connection with the plurality of electrodes G202 and the switch plate G203. In some embodiments, the switch plate G203 is a conductive plate configured to electrically couple at least two conductive posts. Upon proper connection between the switch plate G203 and the conductive posts, the function module (e.g., module 10) and the interface module (e.g., module 20) may be signal communicatively connected, and the functionality of the wearable sensor device may be initiated.

In typical embodiments, the electrodes G202 include a working electrode G202W, a reference electrode G202R, and a counter electrode G202C. In the illustrated embodiment, the working electrode G202R corresponds to a first group of microprobes (e.g., as shown by the group of 4 protruding piercers on the electrode plate G202W). Likewise, the reference electrode G202R corresponds to a second group of microprobes (e.g., as illustrated by the group of 2 protruding piercers on electrode plate G202R arranged on the left hand side of the working electrode G202W). Similarly, the counter electrode G202C corresponds to a third group of microprobes (e.g., the group of 2 probe piercers on electrode plate G202C on the right side of the working electrode G202W).

In the illustrated embodiment, the user interface module further includes a trigger electrode G204. In some embodiments, the trigger electrode corresponds to a fourth group of microprobe (e.g., which comprises 4 probe overlapping the working electrode G202W). The trigger electrode G204 is associated with a trigger circuit (e.g., circuit 302 shown in FIG.21) in the function module configured to generate an alert signal when sensing a drop of contact signal below the predetermined signal level.

In some embodiments, the first, second, and third group of microprobes respectively comprise an array of piercers G202-2 extend from a perforated plate (e.g., plate G202-1). In some embodiments, each microprobe includes only one piercer G202-2. In some other embodiment, each microprobe includes a plurality of piercers G202-2. On the other hand, the fourth group of microprobe G204 includes sensing surfaces G204-3 attached to a perforated plate G204-1. In some embodiments, the sensing surfaces G204-3 are embossed on the perforated plate G204-1.

In some embodiments, the first group of microprobes G202W is disposed between the second group of microprobes G202R and the third group of microprobes G202C. In some embodiments, the fourth group of microprobe G204 is arranged projectively overlap the perforated plate G202-1 of the first group of microprobe G202W. In some embodiments, the perforated plate G202-1 of the first group of microprobe G202W is disposed above the perforated plate G204-1 of the fourth group of microprobe G204. In some embodiments, an insulator G205 is disposed between the perforated plate G202-1 of the first group of microprobe G202W and the perforated plate G204-1 of the fourth group of microprobe G204 to prevent cross talk. In some embodiments, a thickness of the insulator G205 ranges between 2um to 80um. In some embodiments, the through holes of the first group of microprobes G202W, the through holes of the fourth group of microprobe G204, and through holes of the insulator G205 may be aligned with each other.

In some embodiments, the height of the microprobes refers to the height of the piercers G202-2, G204-2. A probe from the first group of microprobes G202W has a first height. A probe from the second group of microprobes G202R has a second height. A probe from the third group of microprobes G202C has a third height. A probe from the fourth group of microprobe G204 has a fourth height. In some embodiments, the first height and the third height are substantially identical. In some embodiments, the second height is substantially the same with the first height and the third height. In some embodiments, the fourth height is less than the first height and the third height. In some embodiments, the fourth height is less than the second height. In the illustrated embodiment, the longer probes G202-2 of probe group G202W, G202R, and G202C correspond respectively to the working, the reference, and the counter electrodes, while the shorter probes G204-3 from the fourth group of microprobes G204 corresponds to the trigger electrode (e.g., associated with a trigger circuit 302 as shown in FIG.21).

Depending on layout arrangement of the microprobe/piercer, the fourth group of microprobe may be of a single piercer configuration (e.g., as illustrated in FIG.11) or of a multi-piercer setup. For instance, the embodiment of FIG.9 incorporates a multi-piercer setup, in which 4 microprobes G204-3 in association with the trigger electrode G204 are arranged to protrude from through holes of the working electrode G202W.

In some embodiments, the first height and the second height are less than 2000um. In some embodiments, the first height and the second height are substantially 1000um. In some embodiments, the fourth group of microprobe G204 has a sensing surface G204-3 substantially planar to the perforated plate G202-1 of the first group of microprobes G202W. In some other embodiments, the fourth group of microprobe G204 has a sensing surface G204-3 is protruding to the perforated plate G202-1 of the first group of microprobes G202W to determine contact of the wearable sensing device when in use.

In some embodiments, the ratio between the number of microprobes in the first group of microprobes G202W and the number of microprobes in the third group of microprobes G202C ranges between 2:1 to 3:1. In some embodiments, the number of microprobes in the first group of microprobes G202W is four and the number of microprobes in the third group of microprobes G202C is two. In some embodiments, the number of microprobes in the first group of microprobes G202W is nine and the number of microprobes in the third group of microprobes G202C is three. In some embodiments, the number of microprobe of the fourth group of microprobe G204 is the same as the number of microprobes in the first group of microprobes G202W. In some other embodiments, the number of microprobe of the fourth group of microprobe G204 is one. In some other embodiments, the number of microprobe of the fourth group of microprobe G204 is greater than one.

In some embodiments, a coupling region G1022-1 of the perforated plates G202-1 of the first, second, third, and fourth group of microprobes are exposed through the back face of the carrier G201. In some embodiments, conductive posts of the function module are correspondingly electrically coupled to the coupling region G1022-1 of the perforated plates G202-1 when the user interface module is detachably joined to the function module.

When the perforated plate G202-1 of the first group of microprobe G202W and the perforated plate G204-1 of the fourth group of microprobe G204 overlap each other, the perforated plate disposed closest to the back face of the carrier G201 is formed to have a notch. In this way, a corresponding conductive post of the function module will be able to electrically couple to the perforated plate disposed farthest from the back face of the carrier G201. In the exemplary embodiment shown in FIG.9, the notch is formed on the perforated plate G204-1 of the fourth group of microprobe G204 since the perforated plate G204-1 is disposed closest to the carrier G201.

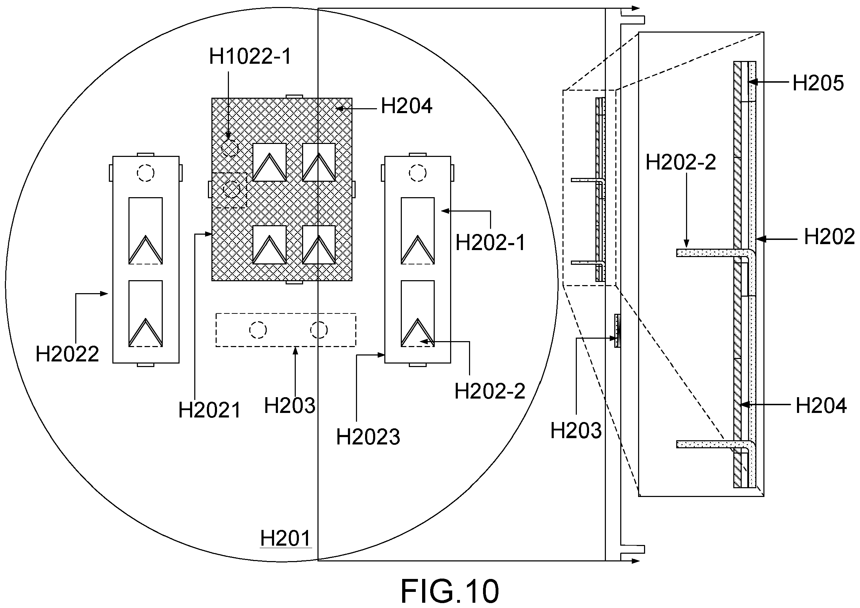

FIG.10 illustrates a planar view of a user interface module of a wearable sensing device according to some embodiments of the present disclosure. In addition, a cross-sectional view (e.g., along a cutline through electrodes H204 and H202W) is also provided in supplement to the overhead illustration.

The user interface module includes a carrier H201, a plurality of biometric sensing electrodes (e.g., H202W/R/C, each comprises an array of probes/piercers H202-2) disposed on a contact face of the carrier H201, and a switch plate H203 disposed on a back face of the carrier H201. As shown in the instant embodiment, the conductive posts of the function module (e.g., module 10 as shown in FIG.1) may respectively establish electrical connection with the plurality of electrodes H202 and the switch plate H203. In some embodiments, the switch plate H203 is a conductive plate configured to electrically couple at least two conductive posts. Upon proper connection between the switch plate H203 and the conductive posts, the function module (e.g., module 10) and the interface module (e.g., module 20) may be signal communicatively connected, and the functionality of the wearable sensor device may be initiated.

In typical embodiments, the electrodes H202 include a working electrode H202W, a reference electrode H202R, and a counter electrode H202C. In the illustrated embodiment, the working electrode H202R corresponds to a first group of microprobes (e.g., as shown by the group of 4 protruding piercers on the electrode plate H202W). Likewise, the reference electrode H202R corresponds to a second group of microprobes (e.g., as illustrated by the group of 2 protruding piercers on electrode plate H202R arranged on the left hand side of the working electrode H202W). Similarly, the counter electrode H202C corresponds to a third group of microprobes (e.g., the group of 2 probe piercers on electrode plate H202C on the right side of the working electrode H202W).

In the illustrated embodiment, the user interface module further includes a trigger electrode H204. In some embodiments, the trigger electrode corresponds to a fourth group of microprobe (e.g., which comprises a probe overlapping the working electrode H202W). The trigger electrode H204 is associated with a trigger circuit (e.g., circuit 302 shown in FIG.21) in the function module configured to generate an alert signal when sensing a drop of contact signal below the predetermined signal level.

In some embodiments, the first, second, and third group of microprobes respectively comprise an array of piercers H202-2 extend from a perforated plate (e.g., plate H202-1). In some embodiments, each microprobe includes only one piercer H202-2. In some other embodiment, each microprobe includes a plurality of piercers H202-2. On the other hand, the fourth group of microprobe H204 is a perforated plate disposed on the perforated plate H202-1 of the first group of microprobes H202W. In some embodiments, the entirety of a surface of the perforated plate of the fourth group of microprobe H204 is used as a sensing area to determine contact of the user interface module to the skin of the user. In some embodiments, the piercers H202-2 of the first group of microprobes H202W are protruding from through holes of the perforated plate of the fourth group of microprobe H204.

In some embodiments, the first group of microprobes H202W is disposed between the second group of microprobes H202R and the third group of microprobes H202C. In some embodiments, the fourth group of microprobe H204 is arranged to projectively overlap the perforated plate H202-1 of the first group of microprobe H202W. In some embodiments, the perforated plate H202-1 of the first group of microprobe H202W is disposed below the perforated plate H204-1 of the fourth group of microprobe H204. In some embodiments, an insulator H205 is disposed between the perforated plate H202-1 of the first group of microprobe H202W and the fourth group of microprobe H204 to prevent cross talk. In some embodiments, a thickness of the insulator H205 ranges between 2um to 80um.

In some embodiments, the height of the microprobes refers to the height of the piercers H202-2. A probe from the first group of microprobes H202W has a first height. In some embodiments, the first height is the height of the first group of microprobes H202W protruding from the perforated plate of the fourth group of microprobe H204. A probe from the second group of microprobes H202R has a second height. A probe from the third group of microprobes H202C has a third height. A probe from the fourth group of microprobe H204 has a fourth height. In some embodiments, the first height and the third height are substantially identical. In some embodiments, the second height is substantially the same with the first height and the third height. In the illustrated embodiment, the longer probes H202-2 of probe group H202W, H202R, and H202C correspond respectively to the working, the reference, and the counter electrodes, while the shorter probes H204-3 from the fourth group of microprobes H204 corresponds to the trigger electrode (e.g., associated with a trigger circuit 302 as shown in FIG.21).

In some embodiments, the first height and the second height are less than 2000um. In some embodiments, the first height and the second height are substantially 1000um. The shorter length of the trigger electrode probes H204-2 allows the group of trigger probes to serve a forerunner for the rest of the functional electrode probes. By way of example, by strategically placing the shorter trigger probe(s) H204-3 over the working electrode H202W, the retention status of the shorter probes may serve as inherent indication for the longer piercers of the functional electrodes. For instance, if the shorter trigger probes H204-3 receive adequate signal strength that indicates proper retention thereof over a wearer's skin, it would naturally imply the proper seating status of the longer micro-piercers (e.g., H202-2).

In some embodiments, the ratio between the number of microprobes in the first group of microprobes H202W and the number of microprobes in the third group of microprobes H202C ranges between 2:1 to 3:1. In some embodiments, the number of microprobes in the first group of microprobes H202W is four and the number of microprobes in the third group of microprobes H202C is two. In some embodiments, the number of microprobes in the first group of microprobes H202W is nine and the number of microprobes in the third group of microprobes H202C is three. In some embodiments, the number of microprobe of the fourth group of microprobe H204 is one. The fourth group of microprobe H204 is a perforated plate having through holes to expose the at least one of the first, second, and third group of microprobes. In some embodiments, a sensing surface of the fourth group of microprobe H204 is the exposed surface of the perforated plate of the fourth group of microprobe H204.

In some embodiments, a coupling region H1022-1 of the perforated plates H202-1 of the first, second, third, and fourth group of microprobes are exposed through the back face of the carrier H201. In some embodiments, conductive posts of the function module are correspondingly electrically coupled to the coupling region H1022-1 of the perforated plates H202-1 when the user interface module is detachably joined to the function module.

When the perforated plate H202-1 of the first group of microprobes H202W and the fourth group of microprobe H204 overlap each other, the perforated plate disposed closest to the back face of the carrier H201 is formed to have a notch. In this way, a corresponding conductive post of the function module will be able to electrically couple to the perforated plate disposed farthest from the back face of the carrier H201. In the exemplary embodiment shown in FIG. 10, the notch is formed on the perforated plate H202-1 of first group of microprobe H202W since the perforated plate H202-1 is disposed closest to the carrier H201.

FIG. 11 illustrates a planar view of a user interface module of a wearable sensing device according to some embodiments of the present disclosure. In addition, a cross-sectional view (e.g., along a cutline through electrodes I204 and I202W) is also provided in supplement to the overhead illustration.

The user interface module includes a carrier I201, a plurality of biometric sensing electrodes (e.g., I202W/R/C, each comprises an array of probes/piercers I202-2) disposed on a contact face of the carrier I201, and a switch plate I203 disposed on a back face of the carrier I201. As shown in the instant embodiment, the conductive posts of the function module (e.g., module 10 as shown in FIG. 1) may respectively establish electrical connection with the plurality of electrodes I202 and the switch plate I203. In some embodiments, the switch plate I203 is a conductive plate configured to electrically couple at least two conductive posts. Upon proper connection between the switch plate I203 and the conductive posts, the function module (e.g., module 10) and the interface module (e.g., module 20) may be signal communicatively connected, and the functionality of the wearable sensor device may be initiated.

In typical embodiments, the electrodes I202 include a working electrode I202W, a reference electrode I202R, and a counter electrode I202C. In the illustrated embodiment, the working electrode I202R corresponds to a first group of microprobes (e.g., as shown by the group of 4 protruding piercers on the electrode plate I202W). Likewise, the reference electrode I202R corresponds to a second group of microprobes (e.g., as illustrated by the group of 2 protruding piercers on electrode plate I202R arranged on the left hand side of the working electrode I202W). Similarly, the counter electrode I202C corresponds to a third group of microprobes (e.g., the group of 2 probe piercers on electrode plate I202C on the right side of the working electrode I202W).

In the illustrated embodiment, the user interface module further includes a trigger electrode I204. In some embodiments, the trigger electrode corresponds to a fourth group of microprobe (e.g., which comprises 1 probe arranged at the central area of the working electrode I202W). The trigger electrode I204 is associated with a trigger circuit (e.g., circuit 302 shown in FIG.21) in the function module configured to generate an alert signal when sensing a drop of contact signal below the predetermined signal level.

In some embodiments, the first, second, and third group of microprobes respectively comprise an array of piercers I202-2 extend from a perforated plate (e.g., plate I202-1). In some embodiments, each microprobe includes only one piercer I202-2. In some other embodiment, each microprobe includes a plurality of piercers I202-2. On the other hand, fourth group of microprobe I204 includes a sensing surface I204-3. In some embodiments, each microprobe includes only one piercer I202-2. In some other embodiment, each microprobe includes a plurality of piercers I202-2.

In some embodiments, the height of the microprobes refers to the height of the piercers I202-2, I204-3. The first group of microprobes I202W have a first height. The second group of microprobes I202R have a second height. The third group of microprobes I202C have a third height. The fourth group of microprobe I204 have a fourth height. In some embodiments, the first height and the third height are the same height. In some embodiments, the second height is the same height with the first height and the third height. In some embodiments, the fourth height is less than the first height and the third height. In some embodiments, the fourth height is less than the second height. In the illustrated embodiment, the longer probes I202-2 of probe group I202W, I202R, and I202C correspond respectively to the working, the reference, and the counter electrodes, while the shorter probes I204-3 from the fourth group of microprobes I204 corresponds to the trigger electrode (e.g., associated with a trigger circuit 302 as shown in FIG.21).

Depending on layout arrangement of the microprobe/piercer, the fourth group of microprobe may be of a single piercer configuration or of a multi-piercer setup. For instance, the embodiment of FIG. 11 incorporates a single probe setup, in which 1 microprobe I204-3 in association with the trigger electrode I204 is arranged at the central area of the working electrode I202W.

In some embodiments, the first height and the second height are less than 2000um. In some embodiments, the first height and the second height are substantially 1000um. In some embodiments, the fourth height is less than 100um. The shorter length of the trigger electrode probes I204-3 allows the group of trigger probes to serve a forerunner for the rest of the functional electrode probes. By way of example, by strategically placing the shorter trigger probe(s) I204-3 around the working electrode I202W, the retention status of the shorter probes may serve as inherent indication for the longer piercers of the functional electrodes. For instance, if the shorter trigger probes I204-3 receive adequate signal strength that indicates proper retention thereof over a wearer's skin, it would naturally imply the proper seating status of the longer micro-piercers (e.g., I202-2).

In some embodiments, the ratio between the number of microprobes in the first group of microprobes I202W and the number of microprobes in the third group of microprobes I202C ranges between 2:1 to 3:1. In some embodiments, the number of microprobes in the first group of microprobes I202W is four and the number of microprobes in the third group of microprobes I202C is two. In some embodiments, the number of microprobes in the first group of microprobes I202W is nine and the number of microprobes in the third group of microprobes I202C is three. In some embodiments, the number of microprobe of the fourth group of microprobe I204 is one. In some other embodiments, the number of microprobe of the fourth group of microprobe I204 is greater than one.

In some embodiments, a coupling region I1022-1 of the perforated plates I202-1 of the first, second, and third group of microprobes are exposed through the back face of the carrier I201. In some embodiments, conductive posts of the function module are correspondingly electrically coupled to the coupling region E1022-1 of the perforated plates I202-1 when the user interface module is detachably joined to the function module. In some embodiments, a backside of the sensing surface I204-3 is exposed through the back face of the carrier I201. In some embodiments, a conductive post of the function module is correspondingly electrically coupled to the backside of the sensing surface I204-3. During application, the sensing surface I204-3 may be in contact with the skin of the user without piercing the skin of the user. The conductive post is configured to apply force to the sensing surface I204-3 and prevent the sensing surface I204-3 from retracting from the skin when in use.

In some embodiments, the first group of microprobes I202W is disposed between the second group of microprobes I202R and the third group of microprobes I202C. In some embodiments, the fourth group of microprobe I204 is arranged projectively offset the perforated plate I202-1 of the first group of microprobe I202W. In some embodiments, the perforated plate I202-1 of the first group of microprobes I202W is formed to have a hole at a central area to accommodate the fourth group of microprobe I204. In some embodiments, the periphery of the fourth group of microprobe I204 is at a distance from the perforated plate I202-1 of the first group of microprobe I202W to prevent cross talk. In some embodiments, a distance between the sides of the fourth group of microprobe I204 and the perforated plate I202-1 of the first group of microprobe I202W ranges between 100um to 2000um.

The microprobes described in accordance with embodiments of the instant disclosure may be made by punching, molding, etching, micromachining, molding, hot/cold forming processes. For instance, the embodiments demonstrated below show a variety of microprobe arrangements generated through metallurgy works such as stamping process on one or more metal sheet. The material for the microprobes may include, but is not limited to, stainless steel, nickel, nickel alloy, titanium, titanium alloy, carbon nanotube, silicon material, or resin. In some embodiments, the microprobes are further coated biocompatible metals (e.g., gold or palladium). Other material that may be used to form the microprobes include polycarbonate, polymethacrylic acid copolymer, ethylene/vinyl acetate copolymer, Teflon (polytetrafluoroethylene), or polyester.

In some embodiments, the microprobes (e.g., the protruding probes as shown in FIGs.1 to 11) are coated with a sensing polymer, such as an antibody, an aptamer, a recombinant monomer, carbohydrate, glucose oxidase or hydroxybutyrate dehydrogenase. In some other embodiments, the microprobes may be further coated with anti-skin allergy drug.

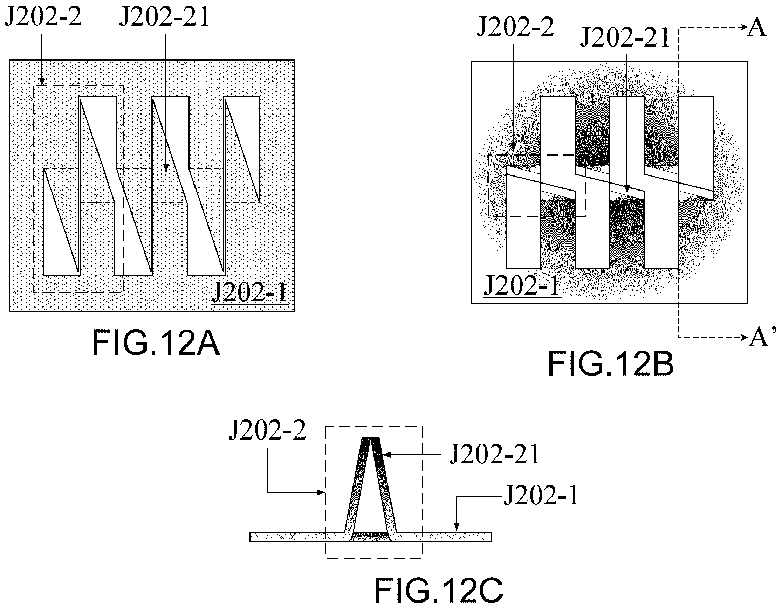

As discussed previously, the adaptation of shorter microprobes may reduce physiological intimidation as well as physical discomfort to a device wearer. Despite the potential delicacy due to inherent limitations from a shorter piercer length, mindful arrangement of the micro-piercers may be employed to further enhance the probing efficiency of the shorter probe in accordance with the instant disclosure. By way of example, in some other embodiment, each microprobe unit may incorporate a plurality of piercers arranged in close proximity configured to induce capillary effect of fluidic analyte upon insertion under a wearer's skin. The induced capillary attraction between micro-piercers may promote extraction of biofluid and in turn enhance sensing efficiency of the wearable device. For example, FIGS. 12A to 20A illustrate planar views of microprobes having a plurality of piercers according to some embodiments of the present disclosure. In the same way as disclosed in the embodiments of FIG.2 to 11, a microprobe having a plurality of piercers may be disposed over the contact face of the carrier of the user interface module. The microprobe having the plurality of piercers may be employed in association with a working electrode, a reference electrode, and a counter electrode.

FIG.12A illustrates a planar view of a microprobe before erecting the microprobe according to some embodiments of the present disclosure. FIG.12B illustrates a planar view of a microprobe after erecting microprobe according to some embodiments of the present disclosure. FIG.12C illustrates a sectional view of a microprobe along line AA' according to some embodiments of the present disclosure.

In some embodiments, a microprobe J202-2 comprises a pair of piercers J202-21 formed on a same perforated plate J202-1. When erected, the piercers J202-21 form a substantially right triangular profile. In some embodiments, the tips of the erected pair of piercers J202-21 does not intersect with each other as shown in FIG. 12C. In some embodiments, a cross section of the pair of piercers J202-21 generates a structural profile that resembles an A-frame. For instance, the piercers J202-21 are similarly sized and arranged in an angle of 20° or less with each other. Moreover, the respective tips to bottoms of the piercers J202-21 cooperatively form a structure similar to an uppercase letter "A". In some other embodiments, a cross sectional profile of the pair of piercers J202-21 form an isosceles triangle. In some embodiments, the ratio of the lengths of the isosceles triangle may be 3:3:1. In some embodiments, the angle formed between the tips of the erected pair of piercers J202-21 are at 20° or less. In some embodiments, the plurality of piercers J202-21 of the plurality of microprobes J202-2 are formed in a continuous array. The plurality of microprobes J202-2 may be formed within one through hole of the perforated plate J202-1 as shown in FIG. 12A.

In some embodiments, the plurality of microprobes J202-2 are coated with a sensing polymer. The sensing polymer may be coated on an outer surface of a piercers J202-21. Further, the sensing polymer may be coated on an inner surface of a piercers J202-21. In this way, when applying the user interface module, at least the sensing polymer coated on an inner surface of a piercers J202-21 are protected from being scraped when penetrating the skin.

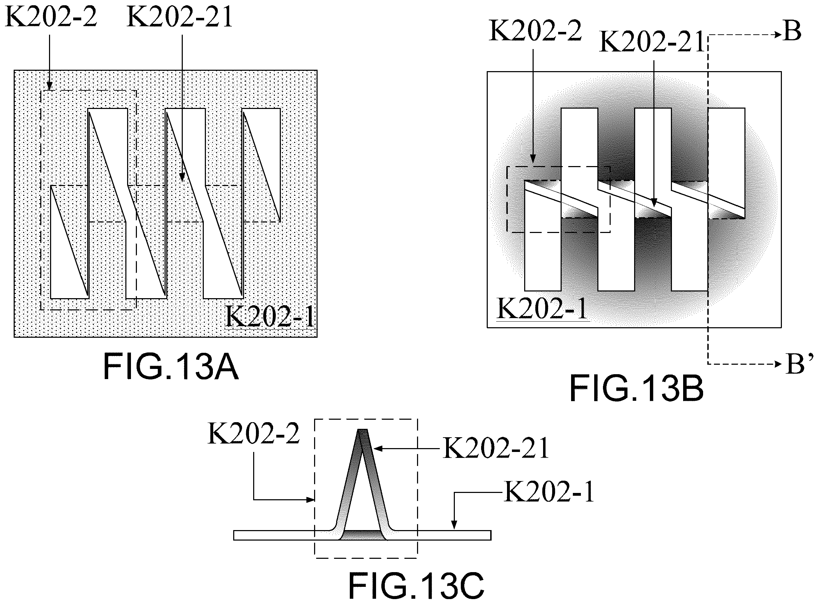

FIG.13A illustrates a planar view of a microprobe before erecting the microprobe according to some embodiments of the present disclosure. FIG.13B illustrates a planar view of a microprobe after erecting microprobe according to some embodiments of the present disclosure. FIG.13C illustrates a sectional view of a microprobe along line BB' according to some embodiments of the present disclosure.

In some embodiments, a microprobe K202-2 comprises a pair of piercers K202-21 formed on a same perforated plate K202-1. When erected, the piercers K202-21 form a substantially right triangular profile In some embodiments, the tips of the erected pair of piercers K202-21 intersect with each other as shown in FIG. 13C to form a pointed tip for penetrating a wearer's skin. In some embodiments, a cross section of the pair of piercers J202-21 generates a structural profile that resembles an A-frame. For instance, the piercers J202-21 are similarly sized and arranged in an angle of 20° or less with each other. Moreover, the respective tips to bottoms of the piercers J202-21 cooperatively form a structure similar to an uppercase letter "A". In some embodiments, a cross sectional profile of the pair of piercers K202-21 form an isosceles triangle. In some embodiments, the ratio of the lengths of the isosceles triangle may be 3:3:1. In some embodiments, the angle formed between the pair of piercers K202-21 are at 20° or less. In some embodiments, the plurality of piercers K202-21 of the plurality of microprobes K202-2 are formed in a continuous array. The plurality of microprobes K202-2 may be formed within one through hole of the perforated plate K202-1 as shown in FIG.13A.

In some embodiments, the plurality of microprobes K202-2 are coated with a sensing polymer. The sensing polymer may be coated on an outer surface of a piercers K202-21. Further, the sensing polymer may be coated on an inner surface of a piercers K202-21. In this way, when applying the user interface module, at least the sensing polymer coated on an inner surface of a piercers K202-21 are protected from being scraped when penetrating the skin.

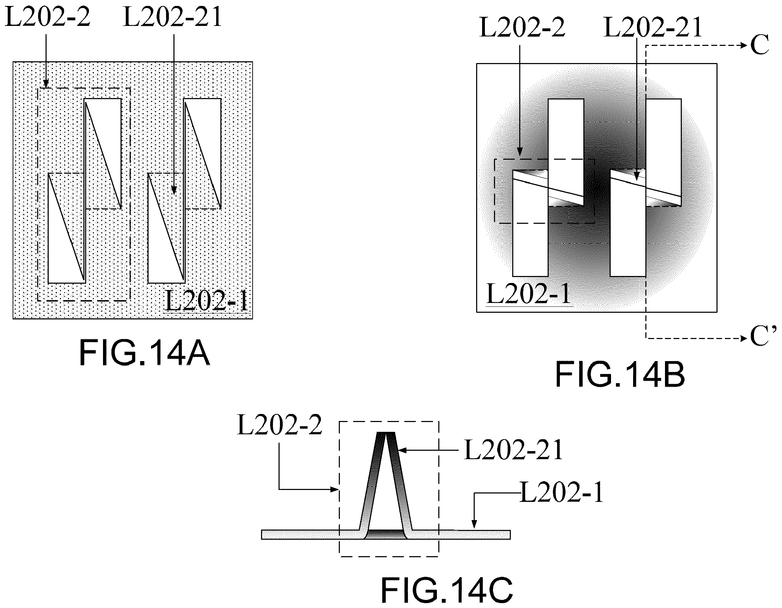

FIG.14A illustrates a planar view of a microprobe before erecting the microprobe according to some embodiments of the present disclosure. FIG.14B illustrates a planar view of a microprobe after erecting microprobe according to some embodiments of the present disclosure. FIG.14C illustrates a sectional view of a microprobe along line CC' according to some embodiments of the present disclosure.

In some embodiments, a microprobe L202-2 comprises a pair of piercers L202-21 formed on a same perforated plate L202-1. When erected, the piercers L202-21 form a profile that is substantially a right triangle. In some embodiments, the tips of the erected pair of piercers L202-21 intersect with each other as shown in FIG. 14C to form a pointed tip for penetrating skin. In some embodiments, a cross sectional profile of the pair of piercers L202-21 form an A-frame. Wherein, the pair of piercers L202-21 are similarly sized, arranged in an angle of 20 degrees or less, and substantially meeting at the tips form a structure similar to an uppercase letter "A". In some embodiments, a cross sectional profile of the pair of piercers L202-21 form an isosceles triangle. The ratio of the lengths of the isosceles triangle may be 3:3:1. In some embodiments, the angle formed between the pair of piercers L202-21 are at 20° or less. In some embodiments, the plurality of microprobes L202-2 are formed in a continuous array having a space between each of the microprobes L202-2. Each microprobe L202-2 may be formed within one through hole of the perforated plate L202-1 as shown in FIG.14A.

In some embodiments, the plurality of microprobes are formed in a continuous array having a space between each of the microprobes. Further, the piercers of each of the microprobes are formed to have a space from each other. Each piercer may be formed within one through hole of the perforated plate.

In some embodiments, the plurality of microprobes L202-2 are coated with a sensing polymer. The sensing polymer is coated on an outer surface of a piercers L202-21. Further, sensing polymer is coated on an inner surface of a piercers L202-21. In this way, when applying the user interface module, at least the sensing polymer coated on an inner surface of a piercers L202-21 are protected from being scraped when penetrating the skin.

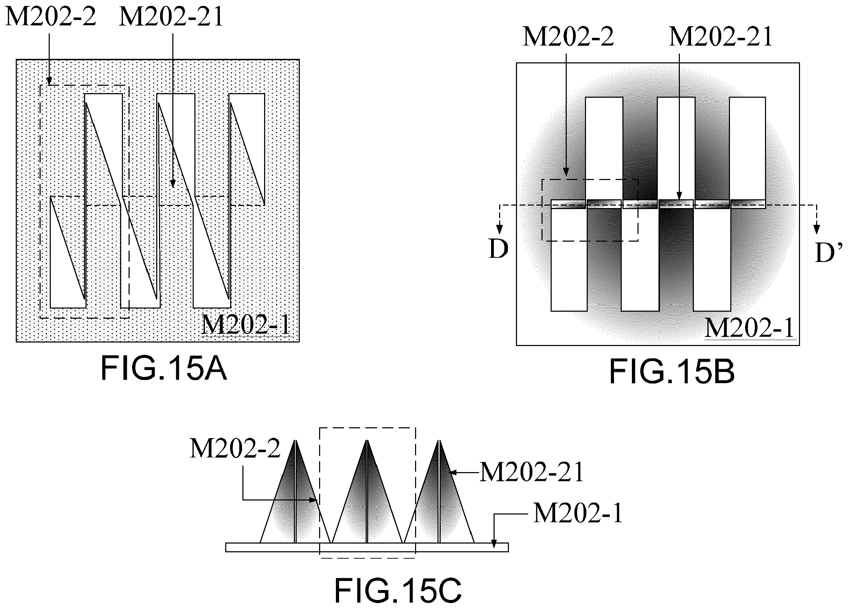

FIG.15A illustrates a planar view of a microprobe before erecting the microprobe according to some embodiments of the present disclosure. FIG.15B illustrates a planar view of a microprobe after erecting microprobe according to some embodiments of the present disclosure. FIG.15C illustrates a sectional view of a microprobe along line DD' according to some embodiments of the present disclosure.

In some embodiments, a microprobe M202-2 comprises a pair of piercers M202-21 formed on a same perforated plate M202-1. When erected, the piercers M202-21 form a profile that is substantially a right triangle. In some embodiments, the erected piercers are substantially perpendicular to a surface of the perforated plate M202-1. In some embodiments, the tips of the erected pair of piercers M202-21 does not intersect with each other as shown in FIG. 15C. In some other embodiments, a cross sectional profile of the pair of piercers M202-21 form an isosceles triangle. In some embodiments, the angle formed between the sides of the erected pair of piercers M202-21 is substantially at 0°. The space between the pair of piercers M202-21 form a capillary tube when inserted into the skin of the user and induces capillary effect. In some embodiments, a width of the space between the pair of piercers M202-21 ranges between 45um to 800um. In some embodiments, the plurality of piercers M202-21 of the plurality of microprobes M202-2 are formed in a continuous array. The plurality of microprobes M202-2 may be formed within one through hole of the perforated plate M202-1 as shown in FIG.15A.

In some embodiments, the plurality of microprobes M202-2 are coated with a sensing polymer. The sensing polymer is coated on an outer surface of a piercers M202-21. As shown in FIG.15B, each of the piercers M202-21 have two outer surfaces. In this way, area sensitive to the target sample is increased.

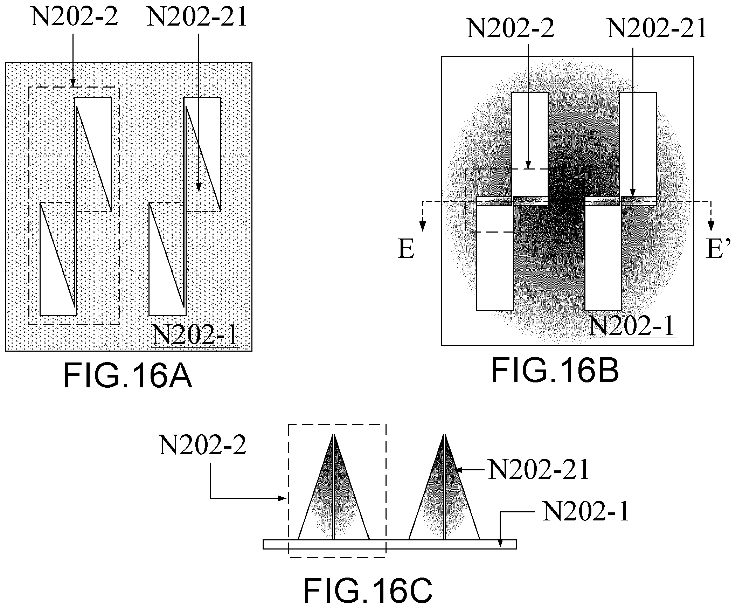

FIG.16A illustrates a planar view of a microprobe before erecting the microprobe according to some embodiments of the present disclosure. FIG.16B illustrates a planar view of a microprobe after erecting microprobe according to some embodiments of the present disclosure. FIG.16C illustrates a sectional view of a microprobe along line EE' according to some embodiments of the present disclosure.

In some embodiments, a microprobe N202-2 comprises a pair of piercers N202-21 formed on a same perforated plate N202-1. When erected, the piercers N202-21 form a profile that is substantially a right triangle. In some embodiments, the erected piercers are substantially perpendicular to a surface of the perforated plate N202-1. In some embodiments, the tips of the erected pair of piercers N202-21 does not intersect with each other as shown in FIG. 16C. In some other embodiments, a cross sectional profile of the pair of piercers N202-21 form an isosceles triangle. In some embodiments, the angle formed between the sides of the erected pair of piercers N202-21 is substantially at 0°. The space between the pair of piercers N202-21 form a capillary tube when inserted into the skin of the user and induces capillary effect. In some embodiments, a width of the gap/space between the neighboring piercer-pair N202-21 ranges from about 45um to about 800um. In some embodiments, the plurality of microprobes N202-2 are formed in a continuous array having a gap between each of the microprobes N202-2. Each microprobe N202-2 may be formed within one through hole of the perforated plate N202-1 as shown in FIG. 16A.

In some embodiments, the plurality of microprobes are formed in a continuous array having a space between each of the microprobes. Further, the piercers of each of the microprobes are formed to have a space from each other. Each piercer may be formed within one through hole of the perforated plate.

In some embodiments, the plurality of microprobes N202-2 are coated with a sensing polymer. The sensing polymer is coated on an outer surface of a piercers N202-21. As shown in FIG. 16B, each of the piercers N202-21 have two outer surfaces. In this way, area sensitive to the target sample is increased.

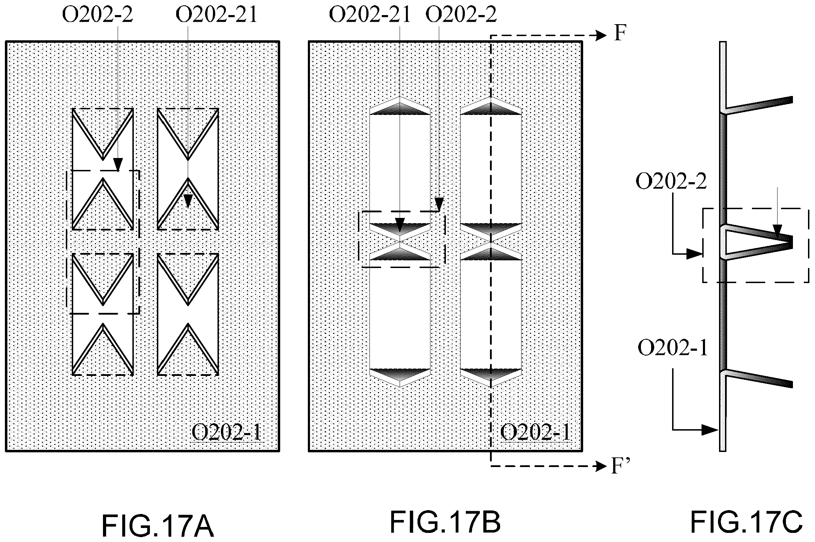

FIG.17A illustrates a planar view of a microprobe before erecting the microprobe according to some embodiments of the present disclosure. FIG.17B illustrates a planar view of a microprobe after erecting microprobe according to some embodiments of the present disclosure. FIG.17C illustrates a sectional view of a microprobe along line FF' according to some embodiments of the present disclosure.

In some embodiments, a microprobe 0202-2 comprises a pair of piercers 0202-21 formed on a same perforated plate 0202-1. When erected, the piercers O202-21 form a profile that is substantially a triangle. In some embodiments, the tips of the erected pair of piercers 0202-21 may or may not be in contact with each other as shown in FIG. 17C. In some embodiments, a cross sectional profile of the pair of piercers 0202-21 form an A-frame. Wherein, the pair of piercers 0202-21 are similarly sized, arranged in an angle of 20 degrees or less, and the tip to bottom form a structure similar to an uppercase letter "A". In some other embodiments, a cross sectional profile of the pair of piercers 0202-21 form an isosceles triangle. The ratio of the lengths of the isosceles triangle may be 3:3:1. In some embodiments, the angle formed between the tips of the erected pair of piercers 0202-21 are at 20° or less.

In some embodiments, the plurality of piercers 0202-21 of the plurality of microprobes O202-2 are formed in a continuous array. The plurality of piercers O202-21 may be formed within one through hole of the perforated plate 0202-1. In an exemplary embodiment shown in FIG. 17A, a pair of piercers 0202-21 are formed within one through hole of the perforated plate 0202-1. The pair of piercers 0202-21 are formed on opposite sides of the through hole of the perforated plate 0202-1. In some embodiments, the piercers 0202-21 formed closest to the periphery of the perforated plate 0202-1 is without a pair to form a triangular cross sectional profile such as shown in FIG. 17C.

In some embodiments, the plurality of microprobes O202-2 are coated with a sensing polymer. The sensing polymer is coated on an outer surface of a piercers 0202-21. Further, sensing polymer is coated on an inner surface of a piercers 0202-21. In this way, when applying the user interface module, at least the sensing polymer coated on an inner surface of a piercers 0202-21 are protected from being scraped when penetrating the skin.

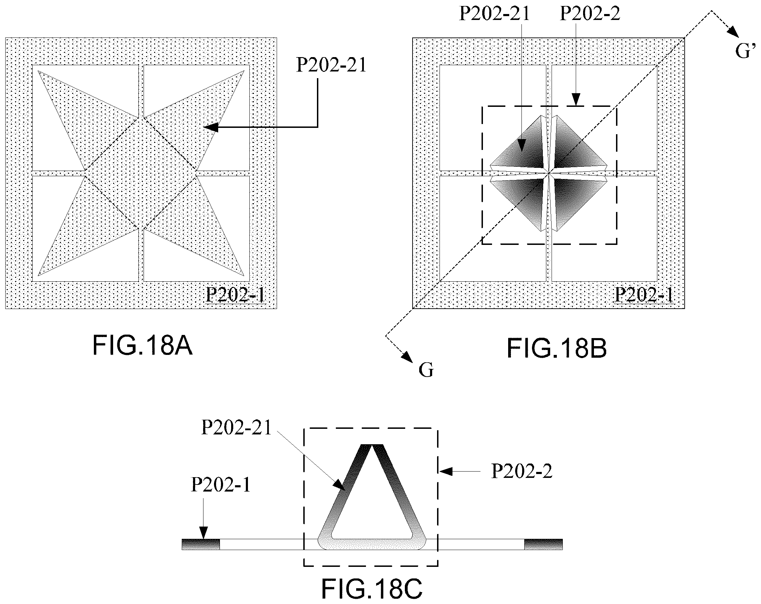

FIG.18A illustrates a planar view of a microprobe before erecting the microprobe according to some embodiments of the present disclosure. FIG.18B illustrates a planar view of a microprobe after erecting microprobe according to some embodiments of the present disclosure. FIG.18C illustrates a sectional view of a microprobe along line GG' according to some embodiments of the present disclosure.

In some embodiments, a microprobe P202-2 comprises a plurality of piercers P202-21 formed on a same perforated plate P202-1. In the exemplary embodiment, the plurality of piercers P202-21 have four piercers P202-21. When erected, the piercers P202-21 form a pyramidal profile. In some embodiments, the tips of the erected piercers P202-21 may or may not be in contact with each other as shown in FIG. 18C. In some embodiments, the plurality of microprobes P202-2 are formed in a continuous array having a space between each of the microprobes P202-2. Further, the piercers P202-21 of each of the microprobes P202-2 are formed to have a space from each other. Each piercer P202-21 may be formed within one through hole of the perforated plate P202-1.

In some embodiments, the plurality of microprobes P202-2 are coated with a sensing polymer. The sensing polymer is coated on an outer surface of a piercers P202-21. Further, sensing polymer is coated on an inner surface of a piercers P202-21. In this way, when applying the user interface module, at least the sensing polymer coated on an inner surface of a piercers P202-21 are protected from being scraped when penetrating the skin.

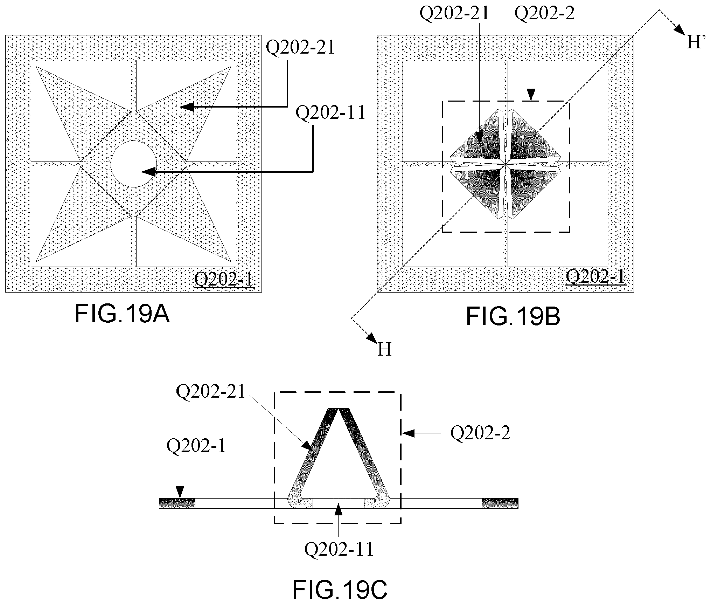

FIG.19A illustrates a planar view of a microprobe before erecting the microprobe according to some embodiments of the present disclosure. FIG.19B illustrates a planar view of a microprobe after erecting microprobe according to some embodiments of the present disclosure. FIG.19C illustrates a sectional view of a microprobe along line HH' according to some embodiments of the present disclosure.

In some embodiments, a microprobe Q202-2 comprises a plurality of piercers Q202-21 formed on a same perforated plate Q202-1. In the exemplary embodiment, the plurality of piercers Q202-21 have four piercers Q202-21. When erected, the piercers Q202-21 form a pyramidal profile. In some embodiments, the tips of the erected piercers Q202-21 may or may not be in contact with each other as shown in FIG. 19C. In some embodiments, the plurality of microprobes Q202-2 are formed in a continuous array having a space between each of the microprobes Q202-2. Further, the piercers Q202-21 of each of the microprobes Q202-2 are formed to have a space from each other. Each piercer Q202-21 may be formed within one through hole of the perforated plate Q202-1. Further, an opening Q202-11 surrounded by the plurality of piercers Q202-21. In some embodiments, the opening Q202-11 can be used to deliver substance into the user when the microprobe Q202-2 is attached to the skin of the user.

In some embodiments, the plurality of microprobes Q202-2 are coated with a sensing polymer. The sensing polymer is coated on an outer surface of a piercers Q202-21. Further, sensing polymer is coated on an inner surface of a piercers Q202-21. In this way, when applying the user interface module, at least the sensing polymer coated on an inner surface of a piercers Q202-21 are protected from being scraped when penetrating the skin.

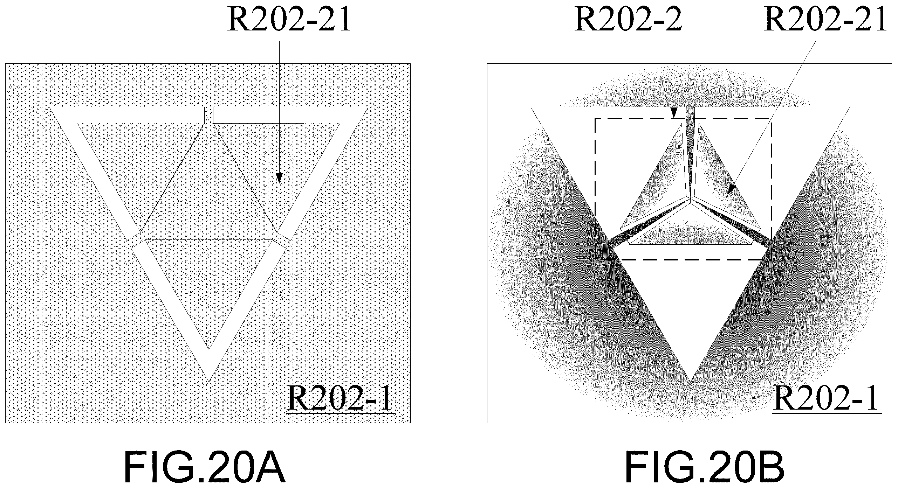

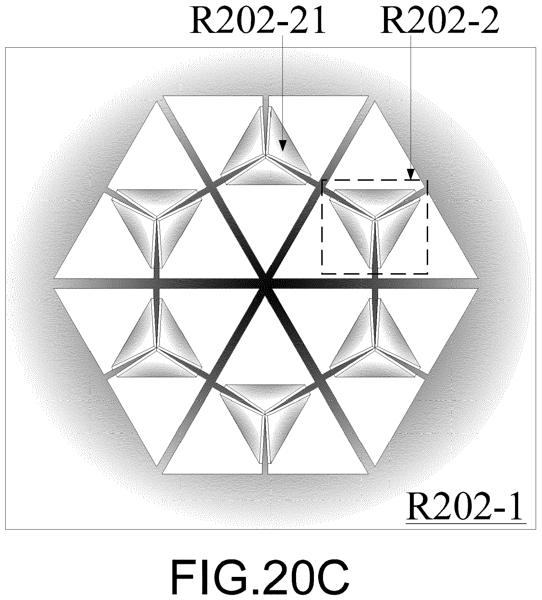

FIG.20A illustrates a planar view of a microprobe before erecting the microprobe according to some embodiments of the present disclosure. FIG.20B illustrates a planar view of a microprobe after erecting microprobe according to some embodiments of the present disclosure. FIG.20C illustrates a planar view of a group of microprobes according to some embodiments of the present disclosure.

In some embodiments, a microprobe R202-2 comprises a plurality of piercers R202-21 formed on a same perforated plate R202-1. In the exemplary embodiment, the plurality of piercers R202-21 have three piercers R202-21. When erected, the piercers R202-21 form a pyramidal profile. In some embodiments, the tips of the erected piercers R202-21 may or may not be in contact with each other. In some embodiments, the plurality of microprobes R202-2 are formed in a continuous array having a space between each of the microprobes R202-2. Further, the piercers R202-21 of each of the microprobes R202-2 are formed to have a space from each other. Each piercer R202-21 may be formed within one through hole of the perforated plate R202-1. In some embodiments, the microprobes R202-2 are formed in an array. In some other embodiments, the microprobes R202-2 are formed annularly as shown in FIG.20C.

In some embodiments, the plurality of microprobes R202-2 are coated with a sensing polymer. The sensing polymer is coated on an outer surface of a piercers R202-21. Further, sensing polymer is coated on an inner surface of a piercers R202-21. In this way, when applying the user interface module, at least the sensing polymer coated on an inner surface of a piercers R202-21 are protected from being scraped when penetrating the skin.

For the microprobes as described in FIGs. 12A to 20A, the material used to form the microprobes include, but is not limited to, stainless steel, nickel, nickel alloy, titanium, titanium alloy, carbon nanotube, silicon material, or resin. In some embodiments, the microprobes are further coated biocompatible metals (e.g., gold or palladium). Other material that may be used to form the microprobes include polycarbonate, polymethacrylic acid copolymer, ethylene/vinyl acetate copolymer, Teflon (polytetrafluoroethylene), or polyester.

In some embodiments, the sensing polymer coated onto the microprobes in FIGs. 12A to 20A includes an antibody, an aptamer, a recombinant monomer, carbohydrate, glucose oxidase and hydroxybutyrate dehydrogenase. In some other embodiments, the microprobes in FIGs. 12A to 20A are further coated with anti-skin allergy drug.

Accordingly, one aspect of the instant disclosure provides a wearable sensing device that comprises a carrier defining a contact face; a first group of microprobes associated with a working electrode protruding from the contact face of the carrier and reaching a first height; a second group of microprobes associated with a counter electrode protruding from the contact face of the carrier and reaching a second height; a third group of microprobes and associated with a reference electrode protruding from the contact face of the carrier and reaching a third height; and a fourth group of microprobe associated with a trigger electrode exposed from the contact face of the carrier and reaching a fourth height. The fourth height is less than the first, second, and third heights.

In some embodiments, the trigger electrode is configured to trigger operation of the working, reference, and counter electrodes upon receiving a contact signal with a predetermined signal level.

In some embodiments, the trigger electrode is associated with a trigger circuit configured to generate an alert signal when sensing a drop of contact signal below the predetermined signal level.

In some embodiments, the first, second, and third heights are less than 2000um and the fourth height is less than 100um.

In some embodiments, a ratio between a number of the first group of microprobes and a number of the second group of microprobes ranges between 2:1 to 3:1.

In some embodiments, the first group of microprobes is formed on a perforated plate. The fourth group of microprobe is arranged projectively offset the perforated plate of the first group of microprobe.

In some embodiments, the fourth group of microprobe is arranged laterally proximate the first group of microprobe, wherein a pitch separation there-between is ranged from about 100um to 2000um.

In some embodiments, the fourth group of microprobe is arranged at periphery of the contact face and around the first group of microprobes.

In some embodiments, the fourth height of the fourth group of microprobe is substantially coplanar to the perforated plate of the first group of microprobes.

In some embodiments, the first group of microprobes is formed on a perforated plate. The fourth group of microprobe is arranged projectively overlap the perforated plate of the first group of microprobe.

In some embodiments, the first group of microprobes is formed on a perforated plate, the fourth group of microprobe is formed on a perforated plate, the perforated plate of the first group of microprobes is stacked over the perforated plate of the fourth group of microprobe.

In some embodiments, an insulator is disposed between the perforated plate of the first group of microprobes and the perforated plate of the fourth group of microprobe.

In some embodiments, the fourth height of the fourth group of microprobe is substantially coplanar to the perforated plate of the first group of microprobe.

Accordingly, another aspect of the instant disclosure provides a wearable sensing system that comprises a user interface module, the wearer interface module including a carrier defining a contact face and a back face opposite the contact face, a first group of microprobes associated with a working electrode protruding from the contact face of the carrier and reaching a first height, a second group of microprobes associated with a reference electrode protruding from the contact face of the carrier and reaching a second height, a third group of microprobes associated with a counter electrode exposed from the contact face of the carrier and reaching a third height, and a fourth group of microprobe associated with a trigger electrode exposed from the contact face of the carrier and reaching a fourth height; and an function module configured to detachably join the user interface module, the function module including a transmitter circuit configured to transmit information gather by the microprobes to an external device. The fourth height is less than the first, second, and third heights.

In some embodiments, the function module further includes conductive posts correspondingly coupling the first, second, third, and fourth group of microprobes to the transmitter circuit.

In some embodiments, the user interface module further includes a switch plate disposed on the back face of the carrier. The switch plate is configured to electrically couple two of the conductive posts to activate the transmitter circuit.

In some embodiments, the conductive posts are spring loaded pins configured to apply constant force while in contact.

In some embodiments, the trigger electrode is configured to trigger operation of the working, reference, and counter electrodes upon receiving a contact signal with a predetermined signal level.

In some embodiments, the trigger electrode is associated with a trigger circuit configured to generate an alert signal when sensing a drop of contact signal below the predetermined signal level.

In some embodiments, the first, second, and third heights are less than 2000um and the fourth height is less than 100um.

In some embodiments, the first group of microprobes is formed on a perforated plate, and the fourth height is substantially planar with the perforated plate.

In some embodiments, a ratio between a number of the first group of microprobes and a number of the second group of microprobes ranges between 2:1 to 3:1.

Accordingly, one aspect of the instant disclosure provides a wearable sensing device that comprises a carrier defining a contact face, a first group of microprobes associated with a working electrode protruding from the contact face of the carrier and reaching a first height, a second group of microprobes associated with a counter electrode protruding from the contact face of the carrier and reaching a second height, and a third group of microprobes associated with a reference electrode exposed from the contact face of the carrier and reaching a third height. The microprobe comprises a plurality of piercers arranged in close proximity and configured to induce capillary attraction of physiological fluid of a wearer.

In some embodiments, the wearable sensing device further comprises a fourth group of microprobe associated with a trigger electrode exposed from the contact face of the carrier and reaching a fourth height; wherein the fourth height is less than the first, second, and third heights.

In some embodiments, the trigger electrode is configured to trigger operation of the working, reference, and counter electrodes upon receiving a contact signal with a predetermined signal level.

In some embodiments, the trigger electrode is associated with a trigger circuit configured to generate an alert signal when sensing a drop of contact signal below the predetermined signal level.

In some embodiments, the plurality of piercers are protruding from a perforated plate, wherein the plurality of piercers comprises two piercers of substantially right triangular profile, wherein corresponding sides of the two piercers substantially perpendicular the perforated plate are arranged substantially parallel to each other.

In some embodiments, a distance between the sides of the two piercers ranges between 45um to 800um.

In some embodiments, the plurality of piercers comprises more than two piercers arranged to form a pyramidal profile.

In some embodiments, the first group of microprobes are coated with an enzyme, the enzyme being coated on an inside surface of the pyramidal shape.

In some embodiments, the piercers with pyramidal profile define an opening that allows feeding of fluid.

In some embodiments, the plurality of piercers comprises two piercers with triangular profile arranged to form an A-frame.

In some embodiments, an angle between the two piercers is no greater than 20 degrees.

In some embodiments, a ratio between a length of the piercers and a distance between bases of the two piercers is substantially 3: 1.

Accordingly, another aspect of the instant disclosure provides a wearable sensing system that comprises a user interface module, the wearer interface module including a carrier defining a contact face and a back face opposite the contact face, a first group of microprobes associated with a working electrode protruding from the contact face of the carrier and reaching a first height, a second group of microprobes associated with a reference electrode protruding from the contact face of the carrier and reaching a second height, and a third group of microprobes associated with a counter electrode exposed from the contact face of the carrier and reaching a third height; and an function module configured to detachably join the user interface module, the function module including a transmitter circuit configured to transmit information gather by the microprobes to an external device. The microprobe comprises a plurality of piercers arranged in close proximity and configured to induce capillary attraction of physiological fluid of a wearer.

In some embodiments, the function module further includes conductive posts correspondingly coupling the first, second, and third group of microprobes to the transmitter circuit.

In some embodiments, the user interface module further includes a switch plate disposed on the back face of the carrier. The switch plate is configured to electrically couple two of the conductive posts to activate the transmitter circuit.

In some embodiments, the conductive posts are spring loaded pins configured to apply constant force while in contact.

In some embodiments, the user interface module further comprises a fourth group of microprobe associated with a trigger electrode protruding from the contact face of the carrier and reaching a fourth height. The fourth height is less than the first, second, and third heights.

In some embodiments, the plurality of piercers are protruding from a perforated plate, wherein the plurality of piercers comprises two piercers of substantially right triangular profile, wherein corresponding sides of the two piercers substantially perpendicular the perforated plate are arranged substantially parallel to each other.

In some embodiments, a distance between the sides of the two piercers ranges between 45um to 800um.

In some embodiments, the plurality of piercers comprises more than two piercers arranged to form a pyramidal profile.

In some embodiments, the first group of microprobes are coated with an enzyme, the enzyme being coated on an inside surface of the pyramidal shape.

In some embodiments, the piercers with pyramidal profile define an opening that allows feeding of fluid.

In some embodiments, the plurality of piercers comprises two piercers with triangular profile arranged to form an A-frame.

In some embodiments, an angle between the two piercers is no greater than 20 degrees.

In some embodiments, a ratio between a length of the piercers and a distance between bases of the two piercers is substantially 3: 1.

Those skilled in the art will readily observe that numerous modifications and alterations of the device and method may be made while retaining the teachings of the invention. Accordingly, the above disclosure should be construed as limited only by the metes and bounds of the appended claims.

## Claims

1. A wearable sensing device, comprising:
a user interface module (20), the user interface module including:
a carrier (A201) defining a contact face;
a first group of microprobes (202W) corresponding to a working electrode protruding from the contact face of the carrier and reaching a first height;
a second group of microprobes (202R) protruding from the contact face of the carrier and reaching a second height;
a third group of microprobes (202C) protruding from the contact face of the carrier and reaching a third height;
a fourth group of microprobes (A204) corresponding to a trigger electrode exposed from the contact face of the carrier and reaching a fourth height;
wherein the second group of microprobes and the third group of microprobes are interchangeably corresponding to a counter electrode and a reference electrode;
wherein the fourth height is less than the first, second, and third heights; and
wherein a trigger circuit (302) in signal communication with the trigger electrode is configured to determine whether the user interface module is correctly attached to the device wearer.

2. The wearable sensing device of claim 1, further comprising:
an function module (10) configured to detachably join the user interface module, the function module including:
a transmitter circuit (202) configured to transmit information gather by the microprobes to an external device.

3. The wearable sensing device of claim 2, wherein the function module (10) further includes:
conductive posts (1022) correspondingly coupling the first, second, third, and fourth group of microprobes to the transmitter circuit;
wherein the user interface module (20) further includes:
a switch plate (203) disposed on the back face of the carrier;
wherein the switch plate is configured to electrically couple two of the conductive posts to activate the transmitter circuit.

4. The wearable sensing device any one of claims 1-3, wherein the trigger circuit (302) is configured to perform a trigger operation of the working, reference, and counter electrodes upon receiving a contact signal with a predetermined signal level.

5. The wearable sensing device of claim 4, wherein the trigger circuit (302) is further configured to generate an alert signal when sensing a drop of contact signal below the predetermined signal level.

6. The wearable sensing device any one of claims 1-5, wherein the first, second, and third heights are less than 2000um and the fourth height is less than 100um.

7. The wearable sensing device any one of claims 1-6, wherein a ratio between a number of the first group of microprobes (202W) and a number of the second group of microprobes (202R) ranges between 2:1 to 3:1.

8. The wearable sensing device any one of claims 1-7, wherein the first group of microprobes (202W) is formed on a perforated plate (A202-1),
wherein the fourth group of microprobes (A204) is arranged projectively offset the perforated plate of the first group of microprobe.

9. The wearable sensing device of claim 8, wherein the fourth group of microprobes (A204) is arranged laterally proximate the first group of microprobes (202W), wherein a pitch separation there-between is ranged from about 100um to 2000um.

10. The wearable sensing device of claim 8, wherein the fourth group of microprobes (A204) is arranged at periphery of the contact face and around the first group of microprobes (202W).

11. The wearable sensing device of claim 8, wherein the fourth group of microprobes (A204) has a sensing surface that is planar to the perforated plate (A202-1) of the first group of microprobes (202W).

12. The wearable sensing device any one of claims 1-7, wherein the first group of microprobes (202W) is formed on a perforated plate (A202-1),
wherein the fourth group of microprobes (A204) is arranged projectively overlap the perforated plate of the first group of microprobe.

13. The wearable sensing device of claim 12, wherein the first group of microprobes (202W) is formed on a perforated plate (A202-1), the fourth group of microprobes (A204) is formed on a perforated plate, the perforated plate of the first group of microprobes is stacked over the perforated plate of the fourth group of microprobe.

14. The wearable sensing device of claim 13, wherein an insulator is disposed between the perforated plate (A202-1) of the first group of microprobes (202W) and the perforated plate of the fourth group of microprobes (A204).

15. The wearable sensing device of claim 12, wherein the fourth group of microprobes (A204) has a sensing surface that is planar to the perforated plate (A202-1) of the first group of microprobe.

## Patentansprüche

1. Erfassende Wearable-Vorrichtung, umfassend:
ein Benutzerschnittstellenmodul (20), wobei das Benutzerschnittstellenmodul einschließt:
einen Träger (A201), der eine Kontaktfläche definiert;
eine erste Gruppe von Mikrosonden (202W), die einer Arbeitselektrode entspricht, die von der Kontaktfläche des Trägers hervorsteht und eine erste Höhe erreicht;
eine zweite Gruppe von Mikrosonden (202R), die von der Kontaktfläche des Trägers hervorsteht und eine zweite Höhe erreicht;
eine dritte Gruppe von Mikrosonden (202C), die von der Kontaktfläche des Trägers hervorsteht und eine dritte Höhe erreicht;
eine vierte Gruppe von Mikrosonden (A204), die einem Starter entspricht, der von der Kontaktfläche des Trägers freiliegt und eine vierte Höhe erreicht;
wobei die zweite Gruppe von Mikrosonden und die dritte Gruppe von Mikrosonden austauschbar einer Gegenelektrode und einer Referenzelektrode entsprechen;
wobei die vierte Höhe kleiner als die erste, die zweite und die dritte Höhe ist; und
wobei eine Auslöseschaltung (302) in Signalkommunikation mit dem Starter konfiguriert ist, um zu bestimmen, ob das Benutzerschnittstellenmodul an dem Vorrichtungstragenden korrekt angebracht ist.

2. Erfassende Wearable-Vorrichtung nach Anspruch 1, ferner umfassend:
ein Funktionsmodul (10), das konfiguriert ist, um sich mit dem Benutzerschnittstellenmodul lösbar zu verbinden, wobei das Funktionsmodul einschließt:
eine Senderschaltung (202), die konfiguriert ist, um Informationen an eine externe Vorrichtung zu senden, die durch die Mikrosonden gesammelt werden.

3. Erfassende Wearable-Vorrichtung nach Anspruch 2, wobei das Funktionsmodul (10) ferner einschließt:
leitfähige Pfosten (1022), die entsprechend die erste, die zweite, die dritte und die vierte Gruppe von Mikrosonden an die Senderschaltung koppeln;
wobei das Benutzerschnittstellenmodul (20) ferner einschließt:
eine Schalterplatte (203), die auf der Rückseite des Trägers eingerichtet ist;
wobei die Schalterplatte konfiguriert ist, um zwei der leitfähigen Pfosten elektrisch zu koppeln, um die Senderschaltung zu aktivieren.

4. Erfassende Wearable-Vorrichtung nach Anspruch 1 bis 3, wobei der Starter (302) konfiguriert ist, um einen Auslösevorgang der Arbeits-, Referenz- und Gegenelektroden bei einem Empfangen eines Kontaktsignals mit einem zuvor bestimmten Signalpegel durchzuführen.

5. Erfassende Wearable-Vorrichtung nach Anspruch 4, wobei die Auslöseschaltung (302) konfiguriert ist, um ein Warnsignal zu erzeugen, wenn ein Abfallen des Kontaktsignals unter den zuvor bestimmten Signalpegel erfasst wird.

6. Erfassende Wearable-Vorrichtung nach einem der Ansprüche 1 bis 5, wobei die erste, die zweite und die dritte Höhe weniger als 2000 um betragen und die vierte Höhe weniger als 100 um beträgt.

7. Erfassende Wearable-Vorrichtung nach einem der Ansprüche 1 bis 6, wobei ein Verhältnis zwischen einer Anzahl der ersten Gruppe von Mikrosonden (202W) und einer Anzahl der zweiten Gruppe von Mikrosonden (202R) zwischen 2 : 1 bis 3 : 1 liegt.

8. Erfassende Wearable-Vorrichtung nach einem der Ansprüche 1 bis 7, wobei die erste Gruppe von Mikrosonden (202W) auf einem Lochblech (A202-1) ausgebildet ist,
wobei die vierte Gruppe von Mikrosonden (A204) dem Lochblech der ersten Gruppe von Mikrosonden vorspringend versetzt angeordnet ist.

9. Erfassende Wearable-Vorrichtung nach Anspruch 8, wobei die vierte Gruppe von Mikrosonden (A204) seitlich nahe der ersten Gruppe von Mikrosonden (202W) angeordnet ist, wobei eine Pitch-Trennung dazwischen von etwa 100 um bis 2000 um liegt.

10. Erfassende Wearable-Vorrichtung nach Anspruch 8, wobei die vierte Gruppe von Mikrosonden (A204) an einem Umfang der Kontaktfläche und um die erste Gruppe von Mikrosonden (202W) herum angeordnet ist.

11. Erfassende Wearable-Vorrichtung nach Anspruch 8, wobei die vierte Gruppe von Mikrosonden (A204) eine Erfassungsoberfläche aufweist, die zu dem Lochblech (A202-1) der ersten Gruppe von Mikrosonden (202W) eben ist.

12. Erfassende Wearable-Vorrichtung nach einem der Ansprüche 1 bis 7, wobei die erste Gruppe von Mikrosonden (202W) auf einem Lochblech (A202-1) ausgebildet ist, wobei die vierte Gruppe von Mikrosonden (A204) angeordnet ist, um das Lochblech der ersten Gruppe von Mikrosonden vorspringend zu überlappen.

13. Erfassende Wearable-Vorrichtung nach Anspruch 12, wobei die erste Gruppe von Mikrosonden (202W) auf einem Lochblech (A202-1) ausgebildet ist, die vierte Gruppe von Mikrosonden (A204) auf einem Lochblech ausgebildet ist, wobei das Lochblech der ersten Gruppe von Mikrosonden über das Lochblech der vierten Gruppe von Mikrosonden gestapelt ist.

14. Erfassende Wearable-Vorrichtung nach Anspruch 13, wobei ein Isolator zwischen dem Lochblech (A202-1) der ersten Gruppe von Mikrosonden (202W) und dem Lochblech der vierten Gruppe von Mikrosonden (A204) eingerichtet ist.

15. Erfassende Wearable-Vorrichtung nach Anspruch 12, wobei die vierte Gruppe von Mikrosonden (A204) eine Erfassungsoberfläche aufweist, die zu dem Lochblech (A202-1) der ersten Gruppe von Mikrosonde eben ist.

## Revendications

1. Dispositif de détection portable, comprenant :
un module d'interface utilisateur (20), le module d'interface utilisateur comportant :
un support (A201) définissant une face de contact ;
un premier groupe de microsondes (202W) correspondant à une électrode de travail faisant saillie à partir de la face de contact du support et atteignant une première hauteur ;
un deuxième groupe de microsondes (202R) faisant saillie à partir de la face de contact du support et atteignant une deuxième hauteur ;
un troisième groupe de microsondes (202C) faisant saillie à partir de la face de contact du support et atteignant une troisième hauteur ;
un quatrième groupe de microsondes (A204) correspondant à une électrode de déclenchement exposée à partir de la face de contact du support et atteignant une quatrième hauteur ;
le deuxième groupe de microsondes et le troisième groupe de microsondes correspondant de manière interchangeable à une contre-électrode et une électrode de référence ;
la quatrième hauteur étant inférieure aux première, deuxième et troisième hauteurs ; et
un circuit de déclenchement (302) en communication de signal avec l'électrode de déclenchement étant configuré pour déterminer si le module d'interface utilisateur est correctement fixé au porteur du dispositif.

2. Dispositif de détection portable selon la revendication 1, comprenant en outre :
un module de fonction (10) configuré pour se raccorder de manière amovible au module d'interface utilisateur, le module de fonction comportant :
un circuit émetteur (202) configuré pour transmettre des informations collectées par les microsondes à un dispositif externe.

3. Dispositif de détection portable selon la revendication 2, dans lequel le module de fonction (10) comporte en outre :
des bornes conductrices (1022) couplant de manière correspondante les premier, deuxième et troisième groupes de microsondes au circuit émetteur ;
le module d'interface utilisateur (20) comportant en outre :
une plaque de commutation (203) disposée sur la face arrière du porteur ;
la plaque de commutation étant configurée pour coupler électriquement deux des bornes conductrices pour activer le circuit émetteur.

4. Dispositif de détection portable selon l'une quelconque des revendications 1 à 3, dans lequel l'électrode de déclenchement (302) est configurée pour effectuer une opération de déclenchement des électrodes de travail, de référence et des contre-électrodes lors de la réception d'un signal de contact avec un niveau de signal prédéterminé.

5. Dispositif de détection portable selon la revendication 4, dans lequel le circuit de déclenchement (302) est en outre configuré pour générer un signal d'alerte lors de la détection d'une chute de signal de contact en dessous du niveau de signal prédéterminé.

6. Dispositif de détection portable selon l'une quelconque des revendications 1 à 5, dans lequel les première, deuxième et troisième hauteurs sont inférieures à 2000 um et la quatrième hauteur est inférieure à 100 um.

7. Dispositif de détection portable selon l'une quelconque des revendications 1 à 6, dans lequel un rapport entre un nombre du premier groupe de microsondes (202W) et un nombre du deuxième groupe de microsondes (202R) est compris entre 2:1 et 3:1.

8. Dispositif de détection portable selon l'une quelconque des revendications 1 à 7, dans lequel le premier groupe de microsondes (202W) est formé sur une plaque perforée (A202-1),
le quatrième groupe de microsondes (A204) étant disposé de manière à être décalé en projection par rapport à la plaque perforée du premier groupe de microsondes.

9. Dispositif de détection portable selon la revendication 8, dans lequel le quatrième groupe de microsondes (A204) est disposé latéralement à proximité du premier groupe de microsondes (202W), une séparation de pas entre eux étant comprise entre environ 100 um et 2000 um.

10. Dispositif de détection portable selon la revendication 8, dans lequel le quatrième groupe de microsondes (A204) est disposé au niveau de la périphérie de la face de contact et autour du premier groupe de microsondes (202W).

11. Dispositif de détection portable selon la revendication 8, dans lequel le quatrième groupe de microsondes (A204) a une surface de détection qui est planaire par rapport à la plaque perforée (A202-1) du premier groupe de microsondes (202W).

12. Dispositif de détection portable selon l'une quelconque des revendications 1 à 7, dans lequel le premier groupe de microsondes (202W) est formé sur une plaque perforée (A202-1), le quatrième groupe de microsondes (A204) étant disposé de manière à chevaucher en projection la plaque perforée du premier groupe de microsondes.

13. Dispositif de détection portable selon la revendication 12, dans lequel le premier groupe de microsondes (202W) est formé sur une plaque perforée (A202-1), le quatrième groupe de microsondes (A204) est formé sur une plaque perforée, la plaque perforée du premier groupe de microsondes est empilée sur la plaque perforée du quatrième groupe de microsondes.

14. Dispositif de détection portable selon la revendication 13, dans lequel un isolant est disposé entre la plaque perforée (A202-1) du premier groupe de microsondes (202W) et la plaque perforée du quatrième groupe de microsondes (A204).

15. Dispositif de détection portable selon la revendication 12, dans lequel le quatrième groupe de microsondes (A204) a une surface de détection qui est planaire par rapport à la plaque perforée (A202-1) du premier groupe de microsondes.
